# FASCICULE DE BREVET EUROPEEN

(11) **EP 3 797 636 B1**
(45) Date de publication et mention de la délivrance du brevet: **07.02.2024**
(21) Numéro de dépôt: 20208138.6
(22) Date de dépôt: 06.06.2013
(51) Int. Cl.: A45D 7/06

(54) **PROCEDE DE MISE EN FORME CAPILLAIRE UTILISANT DES TENSIOACTIFS**
VERFAHREN ZUR KAPILLAREN FORMGEBUNG UNTER VERWENDUNG VON TENSIDEN
METHOD FOR SHAPING HAIR USING SURFACTANTS

(30) Priorité: 07.06.2012 FR 1255307; 07.06.2012 FR 1255309; 07.06.2012 FR 1255310; 15.09.2012 US 201261701648 P; 15.09.2012 US 201261701649 P; 15.09.2012 US 201261701650 P
(43) Date de publication de la demande: 31.03.2021
(62) Demande divisionnaire de: 18176078.6
(73) Titulaire: L'OREAL, 75008 Paris (FR)
(72) Inventeur: VIC, Gabin, 45400 Semoy (FR); CHASY, Maryse, 95190 Goussainville (FR); NUZZO, Stefania, 75014 Paris (FR)
(74) Mandataire: Cabinet Nony

(56) Documents cités:
- FR-A1- 2 959 917

## Description

La présente invention concerne le domaine des procédés de traitement des fibres kératiniques, de préférence les cheveux.

### ARRIERE-PLAN

Les traitements cosmétiques de mise en forme durable des cheveux sont principalement réalisés à l'aide de produits chimiques.

Deux techniques, toutes deux basées sur une rupture des liaisons disulfures - S-S- présentes dans la kératine (cystine), sont généralement utilisées pour obtenir une déformation permanente des cheveux.

La première technique comporte une première étape consistant à ouvrir les liaisons disulfures à l'aide d'une composition comportant un agent réducteur, par exemple de type acide thioglycolique. Cette première étape de réduction est généralement menée à un pH compris entre 8 et 9.

Cette première technique comporte ensuite, de préférence après rinçage des cheveux, une deuxième étape consistant à reconstituer les liaisons disulfures en appliquant sur les cheveux une composition oxydante appelée fixateur. Les cheveux peuvent, préalablement à l'application de la composition réductrice, être mis sous tension par des dispositifs appropriés comme des bigoudis ou être lissés. L'étape d'oxydation peut être en particulier menée à un pH d'environ 3 avec du peroxyde d'hydrogène et peut faciliter la formation de nouveaux ponts disulfure permettant de maintenir la chevelure dans la forme recherchée.

La seconde technique comporte une étape de lanthionisation mettant en oeuvre une composition comportant une base appartenant à la famille des hydroxydes. L'étape de lanthionisation est généralement menée à un pH basique d'environ 13. La lanthionisation est la transformation des ponts disulfures en ponts monosulfures. Ce type de traitement est principalement utilisé pour la mise en forme des cheveux naturellement crépus.

Afin d'obtenir des performances satisfaisantes en termes de durabilité de la mise en forme, les compositions utilisées dans les traitements connus de l'état de la technique peuvent comporter des concentrations relativement importantes en actifs chimiques (agents réducteurs ou composés hydroxydes, par exemple). Ainsi, l'acide thioglycolique peut par exemple être utilisé, dans certaines compositions, à des concentrations massiques comprises entre 6 et 11 % et la soude à 2 %.

Les produits comportant de l'acide thioglycolique sont d'odeur désagréable, laquelle peut être présente lors de l'application et peut, en outre, perdurer sur le cheveu une fois le traitement réalisé.

En outre, les traitements décrits ci-dessus peuvent conduire à une dégradation irréversible du cheveu induite par des modifications des propriétés intrinsèques de la fibre capillaire.

Ces traitements peuvent aussi irriter le cuir chevelu du fait de leur concentration relativement élevée en actifs chimiques.

Il est, par ailleurs, connu d'apporter de la chaleur durant le traitement afin d'activer les processus. Ces technologies peuvent effectivement permettre d'améliorer les performances cosmétiques mais font toujours intervenir des concentrations élevées en actifs chimiques et peuvent donc présenter les mêmes inconvénients que les traitements décrits ci-dessus.

Les documents WO 2002051281, US 20060042649, US 20040250830, WO 2002100210, US 2000680432, US 6079422, US 5988182, US 5819763, US 5773802, US 5676871, JP 09075125, JP 09051813, AU 9664467, US 5494598, EP 197824, US 4710609, US 4743726, US 4952360, US 5030820, US 5286949 décrivent des objets, par exemple des bigoudis, pouvant être chauffés dans un four ménager à micro-ondes, et utilisés sur cheveux humides pour le séchage et la mise en pli.

Le brevet US 3958340 décrit un procédé de séchage rapide de perruques à l'aide d'air chauffé par un rayonnement micro-onde.

La demande US 20070056960 décrit un outil de mise en forme permettant de boucler, lisser et sécher des mèches de cheveux humides à l'aide de micro-ondes.

Le brevet DE 3148538 décrit un outil cylindrique, protégé par une paroi, autour duquel une mèche de cheveux est enroulée. La mèche est séchée et mise en pli à l'aide de micro-ondes appliquées dans l'espace entre le cylindre et la paroi.

On connaît de FR 2 178 049 des dispositifs de dégagement d'énergie électromagnétique dans des matériaux divers.

Par ailleurs, il est connu de FR 2 114 540 et FR 2 118 945 des procédés de chauffage et séchage des cheveux par utilisation d'un rayonnement électromagnétique.

La demande FR 2 959 917 décrit un procédé de traitement des cheveux dans lequel une tension mécanique est appliquée aux cheveux, qui sont alors exposés à des micro-ondes.

Il existe un besoin de disposer de nouveaux procédés, plus efficaces et moins agressifs, de déformation permanente des cheveux.

Il existe notamment un intérêt à disposer de procédés permettant d'obtenir des performances de mise en forme durable améliorées, tout en minimisant l'impact de produits chimiques sur le cheveu et le cuir chevelu.

Il existe aussi un besoin pour bénéficier de nouveaux dispositifs de mise en forme durable des cheveux.

La présente invention vise à répondre à tout ou partie des besoins précités.

Selon un premier aspect, la présente invention concerne un procédé de traitement cosmétique des fibres kératiniques, de préférence les cheveux, comprenant au moins les étapes consistant à :
a) appliquer, sur lesdites fibres kératiniques, une composition contenant au moins une substance choisie parmi les tensioactifs,
b) appliquer une tension mécanique auxdites fibres kératiniques, et
c) exposer lesdites fibres kératiniques sous tension mécanique à des micro-ondes, à une pression allant de 50 000 à 250 000 Pa, en présence d'au moins un solvant sous forme vapeur au contact des fibres kératiniques et sans qu'il y ait séchage complet des fibres kératiniques pendant la totalité de l'exposition aux micro-ondes,

le ou les solvants sous forme vapeur étant entièrement générés par l'évaporation d'au moins un composé présent, avant émission des micro-ondes, au contact des fibres kératiniques,
l'étape a) ayant lieu préalablement à l'étape c),
caractérisé en ce que ladite composition est exempte d'hydroxydes alcalins ou alcalino-terreux à un pH supérieur à 12 ou d'agents réducteurs permettant la rupture des liaisons disulfures.

Dans le présent texte, l'expression solvant est utilisée pour désigner indifféremment un unique solvant ou un mélange de solvants.

Le procédé selon la présente invention peut être mis en oeuvre afin de conduire à un traitement de mise en forme durable moins agressif pour le cuir chevelu et les cheveux. En effet le procédé selon l'invention permet de s'affranchir de l'utilisation de composés agressifs pour le cuir chevelu.

En outre, l'application aux cheveux d'une composition contenant au moins une substance choisie parmi les tensioactifs, ladite composition étant exempte d'hydroxydes alcalins ou alcalino-terreux à un pH supérieur à 12 ou d'agents réducteurs permettant la rupture des liaisons disulfures, de préférence préalablement à l'application d'une tension mécanique, en présence de micro-ondes et d'un solvant sous forme vapeur permet d'obtenir une mise en forme durable et améliorée des cheveux sans qu'il soit nécessaire d'utiliser des agents réducteurs ou des hydroxydes alcalins ou alcalino-terreux.

En particulier, le procédé selon l'invention met en oeuvre en étape a) une composition contenant au moins un tensioactif, ladite composition étant exempte d'hydroxydes alcalins ou alcalino-terreux à un pH supérieur à 12 ou d'agents réducteurs permettant la rupture des liaisons disulfures.

Le présent brevet décrit également un procédé ne relevant pas de l'invention revendiquée, à savoir un procédé de traitement cosmétique des fibres kératiniques, de préférence les cheveux, comprenant au moins les étapes consistant à :
a) appliquer, sur lesdites fibres kératiniques, une composition contenant au moins un corps gras,
b) appliquer une tension mécanique auxdites fibres kératiniques, et
c) exposer lesdites fibres kératiniques sous tension mécanique à des micro-ondes, à une pression allant de 50 000 à 250 000 Pa, en présence d'au moins un solvant sous forme vapeur au contact des fibres kératiniques et sans qu'il y ait séchage complet des fibres kératiniques pendant la totalité de l'exposition aux micro-ondes,

le ou les solvants sous forme vapeur étant entièrement générés par l'évaporation d'au moins un composé présent, avant émission des micro-ondes, au contact des fibres kératiniques,
l'étape a) ayant lieu préalablement à l'étape c).

Le présent brevet décrit également un procédé ne relevant pas de l'invention revendiquée, à savoir un procédé de traitement cosmétique des fibres kératiniques, de préférence les cheveux, comprenant au moins les étapes consistant à :
a) appliquer, sur lesdites fibres kératiniques, une composition contenant au moins un polymère non siliconé,
b) appliquer une tension mécanique auxdites fibres kératiniques, et
c) exposer lesdites fibres kératiniques sous tension mécanique à des micro-ondes, à une pression allant de 50 000 à 250 000 Pa, en présence d'au moins un solvant sous forme vapeur au contact des fibres kératiniques et sans qu'il y ait séchage complet des fibres kératiniques pendant la totalité de l'exposition aux micro-ondes,

le ou les solvants sous forme vapeur étant entièrement générés par l'évaporation d'au moins un composé présent, avant émission des micro-ondes, au contact des fibres kératiniques,
l'étape a) ayant lieu préalablement à l'étape c).

La présente invention vise un procédé de traitement cosmétique des fibres kératiniques, de préférence les cheveux, comprenant au moins les étapes consistant à :
a) appliquer, sur lesdites fibres kératiniques, une composition contenant au moins un tensioactif,
b) appliquer une tension mécanique auxdites fibres kératiniques, et
c) exposer lesdites fibres kératiniques sous tension mécanique à des micro-ondes, à une pression allant de 50 000 à 250 000 Pa, en présence d'au moins un solvant sous forme vapeur au contact des fibres kératiniques et sans qu'il y ait séchage complet des fibres kératiniques pendant la totalité de l'exposition aux micro-ondes,

le ou les solvants sous forme vapeur étant entièrement générés par l'évaporation d'au moins un composé présent, avant émission des micro-ondes, au contact des fibres kératiniques,
l'étape a) ayant lieu préalablement à l'étape c),
caractérisé en ce que ladite composition est exempte d'hydroxydes alcalins ou alcalino-terreux à un pH supérieur à 12 ou d'agents réducteurs permettant la rupture des liaisons disulfures.

Dans une variante particulière de l'invention, le procédé selon l'invention ne comprend pas d'étape d'application sur les fibres kératiniques, de composition(s) comprenant des hydroxydes alcalins ou alcalino-terreux à un pH supérieur à 12 ou des agents réducteurs permettant la rupture des liaisons disulfures.

Le procédé selon l'invention permet l'obtention de la mise en forme souhaitée : bouclage ou lissage. L'effet est obtenu de manière durable pendant plusieurs semaines. Lorsque le procédé selon l'invention est mis en oeuvre pour obtenir un bouclage, le bouclage obtenu est régulier. On observe également un gain de volume de la coiffure.

Les expressions « comportant un(e) », « comprenant un(e) », « contenant un(e) » doivent être comprises comme « comportant au moins un(e) », « comprenant au moins un(e) », « contenant au moins un(e) ».

L'expression « au moins un(e) » est équivalente à l'expression « un(e) ou plusieurs ».

L'expression « compris(e) entre » doit se comprendre comme bornes incluses.

### ETAPE a)

Le procédé cosmétique de traitement des cheveux selon l'invention, comporte notamment une étape a) comprenant l'application d'une composition contenant au moins une substance choisie parmi les tensioactifs, ladite composition étant exempte d'hydroxydes alcalins ou alcalino-terreux à un pH supérieur à 12 ou d'agents réducteurs permettant la rupture des liaisons disulfures.

Avantageusement la composition utilisée à l'étape a) est une composition aqueuse, c'est-à-dire comprenant au moins 5 % en poids d'eau par rapport au poids total de la composition. De préférence la composition comprend de 20 à 99,9 % en poids d'eau par rapport au poids total de la composition.

Selon un mode de réalisation particulier, préféré de l'invention, l'étape a) est préalable à l'étape b).

Selon un autre mode de réalisation particulier de l'invention, l'étape b) est préalable à l'étape a).

Selon un mode de réalisation particulièrement préféré, les étapes sont effectuées dans l'ordre suivant : étape a) puis étape b) puis étape c).

Le procédé selon l'invention ne comprend pas d'étape d'application sur les fibres kératiniques, de composition(s) comprenant des hydroxydes alcalins ou alcalino-terreux à un pH supérieur à 12 ou des agents réducteurs permettant la rupture des liaisons disulfures. La composition de l'étape a) est exempte d'agent(s) réducteur(s) permettant la rupture des liaisons disulfures.

Au sens de l'invention, par « agent réducteur permettant la rupture des liaisons disulfures » on entend un agent choisi parmi les thiols, les sulfites alcalins, les phosphines et les hydrures.

De même, la composition de l'étape a) est exempte d'hydroxydes alcalins ou alcalino-terreux à un pH supérieur à 12.

Par « exempte », on entend une composition comprenant moins de 0,5 % en poids de l'élément considéré, de préférence, moins de 0,1 % en poids par rapport au poids total de la composition, encore mieux ne contient pas du tout l'élément considéré.

### Corps gras

Le présent brevet décrit une variante ne relevant pas de l'invention revendiquée, à savoir une composition de l'étape a) contenant au moins un corps gras. Selon cette variante ne relevant pas de l'invention revendiquée, la composition comprend de préférence de 30 à 95 % en poids d'eau par rapport au poids total de la composition.

De préférence ladite composition comprend au moins un corps gras dans une teneur variant de 0,1% à 95 % en poids par rapport au poids total de la composition.

Le ou lesdits corps gras peu(ven)t être présent(s) dans ladite composition dans une teneur variant de 1 % à 50 %, de préférence de 5 à 30 % en poids par rapport au poids total de la composition.

Par « corps gras », on entend, un composé organique insoluble dans l'eau à température ordinaire (25°C) et à pression atmosphérique (760 mm de Hg) c'est-à-dire de solubilité inférieure à 5 % et de préférence à 1 % encore plus préférentiellement à 0,1 %. Ils présentent dans leur structure au moins un enchaînement d'au moins deux groupements siloxanes ou une chaîne hydrocarbonée comportant au moins 6 atomes de carbone. En outre, les corps gras sont généralement solubles dans des solvants organiques dans les mêmes conditions de température et de pression, comme par exemple le chloroforme, l'éthanol, le benzène, l'huile de vaseline ou le décaméthylcyclopentasiloxane.

Particulièrement le ou les corps gras n'est(ne sont) ni (poly)oxyalkyléné(s) ni (poly)glycérolé(s).

Plus particulièrement, le ou les corps gras est ou sont choisis parmi les hydrocarbures en C₆-C₁₆, les hydrocarbures à plus de 16 atomes de carbone, les huiles non siliconées d'origine animale, les huiles végétales de type triglycérides, les triglycérides synthétiques, les huiles fluorées, les alcools gras, les acides gras non salifiés, les esters d'acide gras et/ou d'alcool gras différents des triglycérides et des cires végétales, les cires non siliconées, les silicones, et leurs mélanges.

Lles alcools, esters et acides gras présentent plus particulièrement un ou plusieurs groupements hydrocarbonés, linéaires ou ramifiés, saturés ou insaturés, comprenant 6 à 30 atomes de carbone, éventuellement substitués, en particulier par un ou plusieurs groupements hydroxyle (en particulier 1 à 4). S'ils sont insaturés, ces composés peuvent comprendre une à trois double-liaisons carbone-carbone, conjuguées ou non.

De préférence le ou les corps gras est(sont) un(des) corps gras non siliconé(s).

En ce qui concerne, les hydrocarbures en C₆-C₁₆, ces derniers sont linéaires, ramifiés, éventuellement cycliques et de préférence les alcanes. A titre d'exemple, on peut citer l'hexane, le dodécane, les isoparaffines comme l'isohexadécane, l'isodécane.

A titre d'huiles hydrocarbonées d'origine animale, on peut citer le perhydrosqualène.

Les huiles triglycérides d'origine végétale ou synthétique sont choisies de préférence parmi les triglycérides liquides d'acides gras comportant de 6 à 30 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol^{®} 810, 812 et 818 par la société Dynamit Nobel, l'huile de jojoba, l'huile de beurre de karité.

Les hydrocarbures linéaires ou ramifiés, d'origine minérale ou synthétique, de plus de 16 atomes de carbone, sont choisis de préférence parmi les huiles de paraffine, la vaseline, l'huile de vaseline, les polydécènes, le polyisobutène hydrogéné tel que Parléam^{®}.

Les huiles fluorées peuvent être choisies parmi le perfluorométhylcyclopentane et le perfluoro-1,3 diméthylcyclohexane, vendus sous les dénominations de "FLUTEC^{®} PC1" et "FLUTEC^{®} PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050^{®}" et "PF 5060^{®}" par la Société 3M, ou encore le bromoperfluorooctyle vendu sous la dénomination "FORALKYL^{®}" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052^{®}" par la Société 3M.

Les alcools gras sont des alcools saturés ou insaturés, linéaires ou ramifiés, et comportant 6 à 30 atomes de carbone et plus particulièrement de 8 à 30 atomes de carbone, parmi ceux-ci on peut citer par exemple l'alcool cétylique, l'alcool stéarylique et leur mélange (alcoolcétylstéarylique ou alcool cétéarylique), l'octyldodécanol, le 2-butyloctanol, le 2-hexyldécanol, le 2-undécylpentadécanol, l'alcool oléique ou l'alcool linoléique.

Les acides gras non salifiés peuvent être les acides carboxyliques saturés ou insaturés et comportent de 6 à 30 atomes de carbone, en particulier de 9 à 30 atomes de carbone. Ils sont choisis plus particulièrement parmi l'acide myristique, l'acide palmitique, l'acide stéarique, l'acide béhénique, l'acide oléïque, l'acide linoléique, l'acide linolénique et l'acide isostéarique.

Ces acides ne sont pas salifiés. Ceci signifie qu'ils sont introduits sous forme d'acides libres et que la composition ne comprend pas d'agent alcalin entrainant leur salification.

Les esters d'acide gras et/ou d'alcool gras, avantageusement différents des triglycérides mentionnés ci-dessus sont les esters de mono ou polyacides aliphatiques saturés ou insaturés, linéaires ou ramifiés en C₁-C₂₆ et de mono ou polyalcools aliphatiques saturés ou insaturés, linéaires ou ramifiés en C₁-C₂₆, le nombre total de carbone des esters étant plus particulièrement supérieur ou égal à 10.

Parmi les monoesters , on peut citer le béhénate de dihydroabiétyle ; le béhénate d'octyldodécyle ; le béhénate d'isocétyle ; le lactate de cétyle ; le lactate d'alkyle en C₁₂-C₁₅ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, de myristyle, de stéaryle, le stéarate d'hexyle, le stéarate de butyle, le stéarate d'isobutyle ; le malate de dioctyle, le laurate d'hexyle, le laurate de 2-hexyldécyle.

Oon peut également utiliser les esters d'acides di ou tricarboxyliques en C₄-C₂₂ et d'alcools en C₁-C₂₂ et les esters d'acides mono di ou tricarboxyliques et d'alcools di, tri, tétra ou pentahydroxy en C₂-C₂₆.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate de propylène glycol ; le dicaprate de propylène glycol, l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle, le dioctanoate de propylène glycol ; le diheptanoate de néopentyl glycol ; le diisanonate de diéthylène glycol ; et les distéarates de polyéthylène glycol.

Parmi les esters cités ci-dessus, on préfère utiliser les palmitates d'éthyle, d'isopropyle, de myristyle, de cétyle, de stéaryle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

La composition peut également comprendre, à titre d'ester gras, des esters et di-esters de sucres d'acides gras en C₆-C₃₀, de préférence en C₁₂-C₂₂. Il est rappelé que l'on entend par « sucre », des composés hydrocarbonés oxygénés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres convenables, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras peuvent être choisis notamment dans le groupe comprenant les esters ou mélanges d'esters de sucres décrits auparavant et d'acides gras en C₆-C₃₀, de préférence en C₁₂-C₂₂, linéaires ou ramifiés, saturés ou insaturés. S'ils sont insaturés, ces composés peuvent comprendre une à trois double-liaisons carbone-carbone, conjuguées ou non.

Les esters peuvent être également choisis parmi les mono-, di-, tri- et tétra-esters, les polyesters et leurs mélanges.

Ces esters peuvent être par exemple des oléate, laurate, palmitate, myristate, béhénate, cocoate, stéarate, linoléate, linolénate, caprate, arachidonates, ou leurs mélanges comme notamment les esters mixtes oléo-palmitate, oléo-stéarate, palmito-stéarate.

Plus particulièrement, on utilise les mono- et di- esters et notamment les mono- ou di-oléate, stéarate, béhénate, oléopalmitate, linoléate, linolénate, oléostéarate de saccharose, de glucose ou de méthylglucose.

On peut citer à titre d'exemple le produit vendu sous la dénomination Glucate^{®} DO par la société Amerchol, qui est un dioléate de méthylglucose.

On peut aussi citer à titre d'exemples d'esters ou de mélanges d'esters de sucre d'acide gras :
- les produits vendus sous les dénominations F160, F140, F110, F90, F70, SL40 par la société Crodesta, désignant respectivement les palmito-stéarates de sucrose formés de 73 % de monoester et 27 % de di- et tri-ester, de 61 % de monoester et 39 % de di-, tri-, et tétra-ester, de 52 % de monoester et 48 % de di-, tri-, et tétra-ester, de 45 % de monoester et 55 % de di-, tri-, et tétra-ester, de 39 % de monoester et 61 % de di-, tri-, et tétra-ester, et le mono-laurate de sucrose;
- les produits vendus sous la dénomination Ryoto Sugar Esters par exemple référencés B370 et correspondant au béhénate de saccharose formé de 20 % de monoester et 80 % de di-triester-polyester;
- le mono-di-palmito-stéarate de sucrose commercialisé par la société Goldschmidt sous la dénomination Tegosoft^{®} PSE.

On peut aussi utiliser des esters d'acides gras et d'alcools gras. On citera par exemple le produit vendu sous la dénomination CRODAMOL MS-PA (MH) par la société CRODA.

La cire ou les cires non siliconées sont choisies notamment, parmi la cire de Carnauba, la cire de Candelila, et la cire d'Alfa, les cires d'hydrocarbures dont la cire de paraffine, l'ozokérite, et la cire microsristalline les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina) ; d'autres cires ou matières premières cireuses sont notamment les cires marines telles que celle vendue par la Société SOPHIM sous la référence M82, les cires de polyéthylène ou de polyoléfines en général.

Les silicones peuvent se présenter sous forme d'huiles, de cires, de résines ou de gommes.

De préférence, les silicones sont choisies parmi les polydialkylsiloxanes, notamment les polydiméthylsiloxanes (PDMS), et les polysiloxanes organo-modifiés comportant au moins un groupement fonctionnel choisi parmi les groupements aminés, les groupements aryle et les groupements alcoxy.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi:
(i) les polydialkylsiloxanes cycliques comportant de 3 à 7, de préférence de 4 à 5 atomes de silicium. Il s'agit, par exemple, de l'octaméthylcyclotétrasiloxane commercialisé notamment sous le nom de VOLATILE SILICONE^{®} 7207 par UNION CARBIDE ou SILBIONE^{®} 70045 V2 par RHODIA, le décaméthylcyclopentasiloxane commercialisé sous le nom de VOLATILE SILICONE^{®} 7158 par UNION CARBIDE, et SILBIONE^{®} 70045 V5 par RHODIA, ainsi que leurs mélanges.

On peut également citer les cyclocopolymères du type diméthylsiloxanes/ méthylalkylsiloxane, tel que la SILICONE VOLATILE^{®} FZ 3109 commercialisée par la société UNION CARBIDE, de formule :

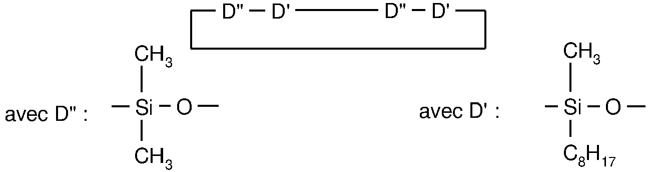

On peut également citer les mélanges de polydialkylsiloxanes cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les polydialkylsiloxanes volatiles linéaires ayant 2 à 9 atomes de silicium et présentant une viscosité inférieure ou égale à 5.10-6m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des polydialkylsiloxanes non volatiles, des gommes et des résines de polydialkylsiloxanes, des polyorganosiloxanes modifiés par les groupements organofonctionnels ci-dessus ainsi que leurs mélanges.

Ces silicones sont plus particulièrement choisies parmi les polydialkylsiloxanes parmi lesquels on peut citer principalement les polydiméthylsiloxanes à groupements terminaux triméthylsilyl. La viscosité des silicones est mesurée à 25 °C selon la norme ASTM 445 Appendice C.

Parmi ces polydialkylsiloxanes, on peut citer à titre non limitatif les produits commerciaux suivants :
- les huiles SILBIONE^{®} des séries 47 et 70 047 ou les huiles MIRASIL^{®} commercialisées par RHODIA telles que, par exemple l'huile 70 047 V 500 000 ;
- les huiles de la série MIRASIL^{®} commercialisées par la société RHODIA ;
- les huiles de la série 200 de la société DOW CORNING telles que la DC200 ayant viscosité 60 000 mm2/s ;
- les huiles VISCASIL^{®} de GENERAL ELECTRIC et certaines huiles des séries SF (SF 96, SF 18) de GENERAL ELECTRIC.

On peut également citer les polydiméthylsiloxanes à groupements terminaux diméthylsilanol connus sous le nom de dimethiconol (CTFA), tels que les huiles de la série 48 de la société RHODIA.

Dans cette classe de polydialkylsiloxanes, on peut également citer les produits commercialisés sous les dénominations "ABIL WAX^{®} 9800 et 9801" par la société GOLDSCHMIDT qui sont des polydialkyl (C1-C20) siloxanes.

Les gommes de silicone sont notamment des polydialkylsiloxanes, de préférence des polydiméthylsiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

Des produits plus particulièrement utilisables sont des mélanges tels que :
- les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthylsiloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10-6m²/s. Ce produit comporte de préférence 15 % de gomme SE 30 et 85 % d'une huile SF96.

Les résines d'organopolysiloxanes sont des systèmes siloxaniques réticulés renfermant les motifs :
R2SiO2/2, R3SiO1/2, RSiO3/2 et SiO4/2
dans lesquelles R représente un alkyl possédant 1 à 16 atomes de carbone. Parmi ces produits, ceux particulièrement préférés sont ceux dans lesquels R désigne un groupe alkyle inférieur en C1-C4, plus particulièrement méthyle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées sont des silicones telles que définies précédemment et comportant dans leur structure un ou plusieurs groupements organofonctionnels fixés par l'intermédiaire d'un groupe hydrocarboné.

Les silicones organomodifiées peuvent être des polydiaryl siloxanes, notamment des polydiphénylsiloxanes, et des polyalkyl-arylsiloxanes fonctionnalisés par les groupes organofonctionnels mentionnés précédemment.

Les polyalkylarylsiloxanes sont particulièrement choisis parmi les polydiméthyl/méthylphénylsiloxanes, les polydiméthyl /diphénylsiloxanes linéaires et/ou ramifiés de viscosité allant de 1.10-5 à 5.10-2m²/s à 25°C.

Parmi ces polyalkylarylsiloxanes, on peut citer à titre d'exemple les produits commercialisés sous les dénominations suivantes :
- les huiles SILBIONE^{®} de la série 70 641 de RHODIA;
- les huiles des séries RHODORSIL^{®} 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Parmi les silicones organomodifiées, on peut aussi citer les polyorganosiloxanes comportant :
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C1-C4 ;
- des groupements alcoxylés, comme le produit commercialisé sous la dénomination "SILICONE COPOLYMER F-755" par SWS SILICONES et ABIL WAX^{®} 2428, 2434 et 2440 par la société GOLDSCHMIDT.

De préférence, le ou les corps gras est(sont) non siliconé(s).

Le ou les corps gras peu(ven)t être choisi(s) parmi les composés liquides ou pâteux à température ambiante (25°C) et à pression atmosphérique.

De préférence, le ou les corps gras est(sont) un(des) composé(s) liquide(s) à température ambiante (25 °C) et à pression atmosphérique.

Encore plus préférentiellement le ou les corps est(sont) liquide(s) et non siliconé(s) à la température de 25 °C et à la pression atmosphérique.

Le ou les corps gras est(sont) avantageusement choisis parmi les hydrocarbures en C6-C16, les hydrocarbures à plus de 16 atomes de carbone, les triglycérides, les alcools gras, les esters d'acide gras et/ou d'alcool gras différents des triglycérides, ou leurs mélanges.

De préférence, le ou les corps gras est ou sont choisis parmi l'huile de vaseline, les polydécènes, les alcools gras liquides, les esters d'acides gras et/ou d'alcools gras liquides, ou leurs mélanges.

Le ou les corps gras peut ou peuvent être choisis parmi les composés solides à température ambiante (25°C) et à pression atmosphérique.

Le ou les corps gras est ou sont avantageusement choisis parmi les cires d'hydrocarbures, les cires végétales, les alcools gras solides, les esters d'acide gras et/ou d'alcool gras solides, ou leurs mélanges.

### Polymères non siliconés

Le présent brevet décrit une variante ne relevant pas de l'invention revendiquée , à savoir une composition de l'étape a) contenant au moins un polymère non siliconé.

Selon cette variante, ne relevant pas de l'invention revendiquée la composition comprend de préférence de 25 à 95 % en poids d'eau, en particulier de 30 à 90 % en poids d'eau par rapport au poids total de la composition.

De préférence ladite composition comprend au moins un polymère non siliconé dans une teneur variant de 0,01 % à 95 % en poids par rapport au poids total de la composition.

Le ou lesdits polymères non siliconés peu(ven)t être présent(s) dans ladite composition dans une teneur variant de 0,1 % à 50 %, de préférence de 1 à 30 % en poids par rapport au poids total de la composition.

Le(s) polymère(s) non siliconé(s) peu(ven)t être choisi(s) parmi les polymères épaississants, fixants ou conditionneurs.

Par « polymère épaississant » on entend un polymère qui, lorsqu'il est introduit à 1 % en poids dans une composition ne le contenant pas, permet d'en augmenter la viscosité d'au moins 100 cps, de préférence au moins 500 cps, à 25 °C et à un taux de cisaillement de 1 s⁻¹. Cette viscosité peut être mesurée à l'aide d'un viscosimètre cône/plan (Rhéomètre Haake R600 ou analogue).

De préférence on utilisera un polymère qui, lorsqu'il est introduit à 1 % en poids dans une solution aqueuse ou hydroalcoolique à 30 % d'éthanol et à pH = 7 ou dans une huile choisie parmi l'huile de vaseline, le myristate d'isopropyle ou le cyclopentadiméthylsiloxane, permet d'atteindre une viscosité d'au moins 100 cps, de préférence au moins 500 cps, à 25 °C et à un taux de cisaillement de 1 s⁻¹. Cette viscosité peut être mesurée à l'aide d'un viscosimètre cône/plan (Rhéomètre Haake R600 ou analogue).

Les polymères épaississants peuvent être épaississants de la phase aqueuse et/ou de la phase grasse, préférentiellement de la phase aqueuse.

Le(s) polymère(s) épaississant(s) utilisé(s) à l'étape a) peu(ven)t être choisi(s) parmi les épaississants cellulosiques notamment l'hydroxyéthycellulose, hydroxypropylcellulose, carboxyméthylcellulose, la gomme de guar et ses dérivés notamment l'hydroxypropylguar, les gommes d'origine microbienne notamment la gomme de xanthane, gomme de scléroglucane, les homopolymères réticulés d'acide acrylique ou d'acide acrylamidopropanesulfonique, les polymères associatifs.

Comme hydroxypropylguar, on citera par exemple le produit vendu par la société RHODIA sous la dénomination commerciale JAGUAR HP 105.

En ce qui concerne les agents épaississants associatifs, on peut mettre en oeuvre un ou plusieurs polymères de nature non ionique ou ionique. De préférence les agents épaississants associatifs sont non ioniques, anioniques ou cationiques.

La structure chimique des polymères associatifs (ou polymères amphiphiles) comprend plus particulièrement au moins une zone hydrophile et au moins une zone hydrophobe. Par groupement hydrophobe, on entend un radical ou polymère à chaîne hydrocarbonée, saturée ou non, linéaire ou ramifiée, comprenant au moins 8 atomes de carbone, de préférence de 10 à 30 atomes de carbone, en particulier de 12 à 30 atomes de carbone et plus préférentiellement de 18 à 30 atomes de carbone.

Préférentiellement, le groupement hydrocarboné provient d'un composé monofonctionnel. A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Parmi les polymères épaississants amphiphiles anioniques comportant au moins une chaîne grasse (hydrophobe) utilisés à l'étape a), on peut citer :
(I) les polymères comportant au moins un motif hydrophile, et au moins un motif éther d'allyle à chaîne grasse, plus particulièrement ceux dont le motif hydrophile est constitué par un monomère anionique insaturé éthylénique, avantageusement par un acide carboxylique vinylique et tout particulièrement par un acide acrylique ou un acide méthacrylique ou les mélanges de ceux ci, et dont le motif éther d'allyle à chaîne grasse correspond au monomère de formule (A) suivante :

   CH2=CR'CH2OBnR (A)
dans laquelle R' désigne H ou CH3, B désigne le radical éthylèneoxy, n est nul ou désigne un entier allant de 1 à 100, R désigne un radical hydrocarboné choisi parmi les radicaux alkyle, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (A) plus particulièrement préféré est un motif dans lequel R' désigne H, n est égal à 10, et R désigne un radical stéaryl (C18).

Parmi ces polymères anioniques à chaîne grasse, on préfère les polymères formés à partir de 20 à 60% en poids d'acide acrylique et/ou d'acide méthacrylique, de 5 à 60% en poids de (méth)acrylates d'alkyles inférieurs, de 2 à 50 % en poids d'éther d'allyl à chaîne grasse de formule (A), et de 0 à 1 % en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC 80 et SALCARE SC90 qui sont des émulsions aqueuses à 30% d'un terpolymère réticulé d'acide méthacrylique, d'acrylate d'éthyle et de steareth-10-allyl éther (40/50/10).
(II) les polymères comportant au moins un motif hydrophile de type acide carboxylique insaturé oléfinique, et au moins un motif hydrophobe de type ester d'alkyl (C10-C30) d'acide carboxylique insaturé.

De préférence, ces polymères sont choisis parmi ceux dont le motif hydrophile de type acide carboxylique insaturé oléfinique correspond au monomère de formule (B) suivante : 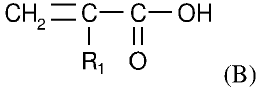
dans laquelle, R1 désigne H ou CH3 ou C2H5, c'est-à-dire des motifs acide acrylique, acide méthacrylique ou acide éthacrylique, et dont le motif hydrophobe de type ester d'alkyl (C10-C30) d'acide carboxylique insaturé correspond au monomère de formule (C) suivante : 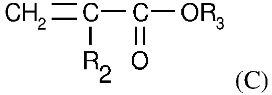
dans laquelle, R2 désigne H ou CH3 ou C2H5 (c'est-à-dire des motifs acrylates, méthacrylates ou éthacrylates) et de préférence H (motifs acrylates) ou CH3 (motifs méthacrylates), R3 désignant un radical alkyle en C10-C30, et de préférence en C12-C22.

Les esters d'alkyles (C10-C30) d'acides carboxyliques insaturés sont par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.

Parmi ce type de polymères anioniques à chaîne grasse, on utilisera plus particulièrement des polymères formés à partir d'un mélange de monomères comprenant :
(i) essentiellement de l'acide acrylique,
(ii) un ester de formule (C) décrite ci-dessus et dans laquelle R2 désigne H ou CH3, R3 désignant un radical alkyle ayant de 12 à 22 atomes de carbone,
(iii) et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ce type de polymères anioniques à chaîne grasse, on utilisera plus particulièrement ceux constitués de 95 à 60 % en poids d'acide acrylique (motif hydrophile), 4 à 40 % en poids d'acrylate d'alkyles en C10-C30 (motif hydrophobe), et 0 à 6 % en poids de monomère polymérisable réticulant, ou bien ceux constitués de 98 à 96 % en poids d'acide acrylique (motif hydrophile), 1 à 4 % en poids d'acrylate d'alkyles en C10-C30 (motif hydrophobe), et 0,1 à 0,6 % en poids de monomère polymérisable réticulant tel que ceux décrits précédemment.

Parmi lesdits polymères ci-dessus, on préfère tout particulièrement selon la présente invention, le produit carbomère vendu par la société LUBRIZOL sous la dénomination CARBOPOL ULTREZ 10, ainsi que les produits vendus par la société GOODRICH sous les dénominations commerciales PEMULEN TR1, PEMULEN TR2, CARBOPOL 1382, et encore plus préférentiellement le PEMULEN TR1, et le produit vendu par la société S.E.P.P.I.C. sous la dénomination COATEX SX.
(III) les terpolymères d'anhydride maléique/α-oléfine en C30-C38/ maléate d'alkyle tel que le produit (copolymère anhydride maléique/α-oléfine en C30-C38/maléate d'isopropyle) vendu sous le nom PERFORMA V 1608 par la société NEWPHASE TECHNOLOGIES.
(IV) les terpolymères acryliques comprenant :
   (a) 20 à 70 % en poids d'un acide carboxylique à insaturation α,β-monoéthylénique,
   (b) 20 à 80 % en poids d'un monomère à insaturation α,β-monoéthylénique non-tensio-actif différent de (a),
   (c) 0,5 à 60 % en poids d'un mono-uréthane non-ionique qui est le produit de réaction d'un tensio-actif monohydrique avec un monoisocyanate à insaturation monoéthylénique,
      tels que ceux décrits dans la demande de brevet EP-A-0173109 et plus particulièrement un terpolymère acide méthacrylique /acrylate de méthyle/diméthyl métaisopropényl benzyl isocyanate d'alcool béhényle éthoxylé (40OE) en dispersion aqueuse à 25%.
-(V) les copolymères comportant parmi leurs monomères un acide carboxylique à insaturation α,β-monoéthylénique et un ester d'acide carboxylique à insaturation α,β-monoéthylénique et d'un alcool gras (C8-C30)oxyalkyléné.

Préférentiellement ces composés comprennent également comme monomère un ester d'acide carboxylique à insaturation α,β-monoéthylénique et d'alcool en C1-C4.

A titre d'exemple de ce type de composé on peut citer l'ACULYN 22 vendu par la société ROHM et HAAS, qui est un terpolymère acide méthacrylique/acrylate d'éthyle/méthacrylate de stéaryle oxyalkyléné.

Le(s) polymère(s) épaississant(s) amphiphile(s) non ionique(s) à chaîne grasse (hydrophobe) pouvant être utilisé(s) à l'étape a) est(sont) choisi(s) de préférence parmi :
(1) les celluloses modifiées par des groupements comportant au moins une chaîne grasse, comme notamment ;
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C8-C22, comme le produit NATROSOL PLUS GRADE 330 CS (alkyles en C16) vendu par la société AQUALON, ou le produit BERMOCOLL EHM 100 vendu par la société BEROL NOBEL,
   - celles modifiées par des groupes polyalkylène glycol éther d'alkyl phénol, tel que le produit AMERCELL POLYMER HM-1500 (polyéthylène glycol (15) éther de nonyl phénol) vendu par la société AMERCHOL.
(2) les hydroxypropylguars modifiés par des groupements comportant au moins une chaîne grasse tel que le produit ESAFLOR HM 22 (chaîne alkyle en C22) vendu par la société LAMBERTI, les produits RE210-18 (chaîne alkyle en C14) et RE205-1 (chaîne alkyle en C20) vendus par la société RHONE POULENC.
(3) les amidons chimiquement modifiés ou non, en particulier les phosphates de diamidon et les carboxyméthylamidon.
(4) les copolymères de vinyl pyrrolidone et de monomères hydrophobes à chaîne grasse,
   avec par exemple :
   - les produits ANTARON V216 ou GANEX V216 (copolymère vinylpyrrolidone / hexadécène) vendu par la société I.S.P.
   - les produits ANTARON V220 ou GANEX V220 (copolymère vinylpyrrolidone / eicosène) vendu par la société I.S.P.
(5) les copolymères de méthacrylates ou d'acrylates d'alkyles en C1-C6 et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208.
(6) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
(7) les polymères à squelette aminoplaste éther possédant au moins une chaîne grasse, tels que les composés PURE THIX proposés par la société SUD-CHEMIE.
(8) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

De préférence, les polyéthers polyuréthanes comportent au moins deux chaînes grasses hydrocarbonées, ayant de 8 à 30 atomes de carbone, séparées par une séquence hydrophile, les chaînes hydrocarbonées pouvant être des chaînes pendantes ou des chaînes en bout de séquence hydrophile.

A titre d'exemples de polyéthers polyuréthanes non ioniques à chaîne grasse utilisables dans l'étape a) de l'invention, on peut utiliser le Rhéolate 205 à fonction urée vendu par la société RHEOX ou encore les Rhéolates 208 , 204 ou 212, ainsi que l'Acrysol RM 184, l'Aculyn ou Acrysol 44 et l'Aculyn ou Acrysol 46 de la société ROHM & HAAS [l'ACULYN 46 est un polycondensat de polyéthylèneglycol à 150 ou 180 moles d'oxyde d'éthylène, d'alcool stéarylique et de méthylène bis(4-cyclohexyl-isocyanate) (SMDI), à 15 % en poids dans une matrice de maltodextrine (4 %) et d'eau (81 %); l'ACULYN 44 est un polycondensat de polyéthylèneglycol à 150 ou 180 moles d'oxyde d'éthylène, d'alcool décylique et de méthylène bis(4-cyclohexylisocyanate) (SMDI), à 35 % en poids dans un mélange de propylèneglycol (39 %) et d'eau (26 %)].

On peut également citer le produit ELFACOS T210 à chaîne alkyle en C12-C 14 et le produit ELFACOS T212 à chaîne alkyle en C18 de chez AKZO, ainsi que le produit DW 1206B de chez ROHM & HAAS à chaîne alkyle en C20 et à liaison uréthanne, proposé à 20 % en matière sèche dans l'eau.

On peut aussi utiliser des solutions ou dispersions de ces polymères notamment dans l'eau ou en milieu hydroalcoolique. A titre d'exemple, de tels polymères on peut citer, le Rhéolate 255, le Rhéolate 278 et le Rhéolate 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Le(s) polymère(s) amphiphile(s) cationique(s) comportant au moins une chaîne grasse (hydrophobe) utilisé(s) à l'étape a) peu(ven)t notamment être choisi(s) parmi les dérivés de cellulose quaternisée, les polyuréthanes cationiques, les polyvinyllactames cationiques, et de préférence, parmi les dérivés de cellulose quaternisée.

A titre d'exemple de polymères de ce type, on peut citer en particulier :
- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

Les radicaux alkyles portés par les celluloses ou les hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthylcelluloses quaternisées à chaînes grasses en C8-C30, les produits QUATRISOFT LM 200, QUATRISOFT LM-X 529-18-A, QUATRISOFT LM-X 529-18B (alkyle en C12) et QUATRISOFT LM-X 529-8 (alkyle en C18) commercialisés par la société AMERCHOL et les produits CRODACEL QM, CRODACEL QL (alkyle en C12) et CRODACEL QS (alkyle en C18) commercialisés par la société CRODA.

Tous les polymères fixants anioniques, cationiques, amphotères, non ioniques et leurs mélanges utilisés dans la technique peuvent être utilisés dans la composition de l'étape a) selon la présente demande.

On entend par polymère fixant tout polymère apte à conférer une forme à une chevelure ou à maintenir une chevelure dans une forme donnée.

Les polymères fixants peuvent être solubles dans le milieu cosmétiquement acceptable ou insolubles dans ce même milieu et utilisés dans ce cas sous forme de dispersions de particules solides ou liquides de polymère (latex ou pseudolatex).

Le(s) polymère(s) fixant(s) anionique(s) généralement utilisé(s) est(sont) un(des) polymère(s) comportant des groupements dérivés d'acide carboxylique, sulfonique ou phosphorique et a(ont) une masse moléculaire moyenne en nombre comprise entre environ 500 et 5 000 000.

Les groupements carboxyliques sont apportés par des monomères mono ou diacides carboxyliques insaturés tels que ceux répondant à la formule (I) : 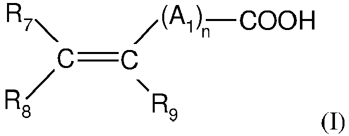 dans laquelle n est un nombre entier de 0 à 10, A1 désigne un groupement méthylène, éventuellement relié à l'atome de carbone du groupement insaturé ou au groupement méthylène voisin lorsque n est supérieur à 1, par l'intermédiaire d'un hétéroatome tel qu'oxygène ou soufre, R7 désigne un atome d'hydrogène, un groupement phényle ou benzyle, R8 désigne un atome d'hydrogène, un groupement alkyle inférieur ou carboxyle, R9 désigne un atome d'hydrogène, un groupement alkyle inférieur, un groupement -CH2-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques préférés selon l'invention sont :
A) Les copolymères d'acide acrylique ou méthacrylique ou leurs sels et d'acrylamide vendus sous la forme de leurs sels de sodium sous les dénominations RETEN 421, 423 ou 425 par la Société HERCULES,
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevet luxembourgeois nos 75370 et 75371 ou proposés sous la dénomination QUADRAMER par la société AMERICAN CYANAMID. On peut également citer les copolymères d'acide acrylique et de méthacrylate d'alkyle en C1-C4 et les terpolymères de vinylpyrrolidone, d'acide acrylique et de méthacrylate d'alkyle en C1-C20, par exemple, de lauryle, tels que celui commercialisé par la société ISP sous la dénomination ACRYLIDONE^{®} LM et les terpolymères acide méthacrylique/acrylate d'éthyle/acrylate de tertiobutyle tels que le produit commercialisé sous la dénomination LUVIMER^{®} 100 P par la société BASF ;
   On peut aussi citer les copolymères acide méthacrylique/ acide acrylique/acrylate d'éthyle/méthacrylate de méthyle en dispersion aqueuse, commercialisé sous la dénomination AMERHOLD^{®} DR 25 par la société AMERCHOL ;
C) Les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique α- ou β-cyclique. De tels polymères sont décrits entre autres dans les brevets français nos 1 222 944, 1 580 545, 2 265 782, 2 265 781, 1 564 110 et 2 439 798. Des produits commerciaux entrant dans cette classe sont les résines 28-29-30, 26-13-14 et 28-13-10 commercialisées par la société National Starch ;
D) Les copolymères d'acides ou d'anhydrides carboxyliques monoinsaturés en C4-C8 choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US nos 2 047 398, 2 723 248, 2 102 113, le brevet GB n° 839 805. Des produits commerciaux sont notamment ceux vendus sous les dénominations GANTREZ^{®} AN ou ES par la société ISP ;
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne,
   - les fonctions anhydrides de ces copolymères étant éventuellement monoestérifiées ou monoamidifiées.

Ces polymères sont par exemple décrits dans les brevets français nos 2 350 384 et 2 357 241 de la demanderesse.
E) Les polyacrylamides comportant des groupements carboxylates.

Les homopolymères et copolymères comprenant des groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone ;
- les sels de l'acide polystyrène-sulfonique tels que les sels de sodium vendus par exemple sous la dénomination Flexan^{®} 500 et Flexan^{®} 130 par National Starch. Ces composés sont décrits dans le brevet FR 2 198 719 ;
- les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER HSP 1180 par HENKEL.

Comme autre polymère fixant anionique utilisable selon l'invention, on peut citer le polymère anionique séquencé branché vendu sous la dénomination Fixate G-100 par la société NOVEON.

Le(s) polymère(s) fixant(s) anionique(s) utilisé(s) à l'étape a) est(sont) de préférence choisi(s) parmi les copolymères d'acide acrylique tels que les terpolymères acide acrylique/acrylate d'éthyle/N-tertiobutylacrylamide vendus notamment sous la dénomination ULTRAHOLD^{®} STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/ néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifiés vendus, par exemple, sous la dénomination GANTREZ^{®} par la société ISP, les copolymères d'acide méthacrylique et de méthacrylate de méthyle vendus sous la dénomination EUDRAGIT^{®} L par la société ROHM PHARMA, les copolymères d'acide méthacrylique et d'acrylate d'éthyle vendus sous la dénomination LUVIMER^{®} MAEX ou MAE par la société BASF et les copolymères acétate de vinyle/acide crotonique vendus sous la dénomination LUVISET CA 66 par la société BASF et les copolymères acétate de vinyle/acide crotonique greffés par du polyéthylèneglycol vendus sous la dénomination ARISTOFLEX^{®} A par la société BASF, le polymère vendu sous la dénomination Fixate G-100 par la société NOVEON.

Parmi les polymères fixants anioniques cités ci-dessus, on préfère plus particulièrement utiliser dans le cadre de la présente invention, les copolymères méthylvinyléther/anhydride maléique monoestérifiés vendus sous la dénomination GANTREZ^{®} ES 425 par la société ISP, les terpolymères acide acrylique/acrylate d'éthyle/N-tertiobutylacrylamide vendus sous la dénomination ULTRAHOLD^{®} STRONG par la société BASF, les copolymères d'acide méthacrylique et de méthacrylate de méthyle vendus sous la dénomination EUDRAGIT^{®} L par la société ROHM PHARMA, les terpolymères acétate de vinyle/tertio-butylbenzoate de vinyle/acide crotonique et les terpolymères acide crotonique/acétate de vinyle/ néododécanoate de vinyle vendus sous la dénomination Résine 28-29-30 par la société NATIONAL STARCH, les copolymères d'acide méthacrylique et d'acrylate d'éthyle vendus sous la dénomination LUVIMER^{®} MAEX OU MAE par la société BASF, les terpolymères vinylpyrrolidone/acide acrylique/méthacrylate de lauryle vendus sous la dénomination ACRYLIDONE^{®} LM par la société ISP, le polymère vendu sous la dénomination Fixate G-100 par la société NOVEON.

Le(s) polymère(s) filmogène(s) fixant(s) cationique(s) utilisable(s) à l'étape a) est(sont) de préférence choisi(s) parmi les polymères comportant des groupements amines primaires, secondaires, tertiaires et/ou quaternaires faisant partie de la chaîne polymère ou directement reliés à celle-ci, et ayant un poids moléculaire compris entre 500 et environ 5 000 000 et de préférence entre 1 000 et 3 000 000.

Parmi ces polymères, on peut citer plus particulièrement les polymères cationiques suivants:
(1) les homopolymères ou copolymères dérivés d'esters ou d'amides acryliques ou méthacryliques et comportant au moins un des motifs de formules suivantes: 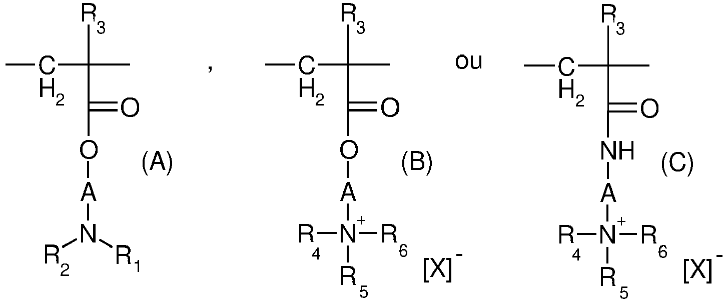 dans lesquelles:
   R3 désigne un atome d'hydrogène ou un radical CH3;
   A est un groupe alkyle linéaire ou ramifié comportant de 1 à 6 atomes de carbone ou un groupe hydroxyalkyle comportant de 1 à 4 atomes de carbone ;
   R4, R5, R6, identiques ou différents, représentent un groupe alkyle ayant de 1 à 18 atomes de carbone ou un radical benzyle;
   R1 et R2, identiques ou différents, représentent chacun un atome hydrogène ou un groupe alkyle ayant de 1 à 6 atomes de carbone;
   X désigne un anion méthosulfate ou un halogénure tel que chlorure ou bromure.

Les copolymères de la famille (1) contiennent en outre un ou plusieurs motifs dérivant de comonomères pouvant être choisis dans la famille des acrylamides, méthacrylamides, diacétones-acrylamides, acrylamides et méthacrylamides substitués sur l'azote par des groupes alkyles inférieurs (C1-C4), des groupes dérivés des acides acryliques ou méthacryliques ou leurs esters, des vinyllactames tels que la vinylpyrrolidone ou le vinylcaprolactame, d'esters vinyliques.

Ainsi, parmi ces copolymères de la famille (1), on peut citer :
- les copolymères d'acrylamide et de méthacrylate de diméthylaminoéthyle quaternisé au sulfate de diméthyle ou avec un halogénure de diméthyle, tels que celui vendu sous la dénomination HERCOFLOC^{®} par la société HERCULES,
- les copolymères d'acrylamide et de chlorure de méthacryloyloxyéthyl-triméthylammonium décrit par exemple dans la demande de brevet EP-A-080976 et vendus sous la dénomination BINA QUAT P 100 par la société CIBA GEIGY,
- le copolymère d'acrylamide et de méthosulfate de méthacryloyloxy-éthyl-triméthyl-ammonium tel que celui vendu sous la dénomination RETEN par la société HERCULES,
- les copolymères vinylpyrrolidone/acrylate ou méthacrylate de dialkyl-amino-alkyle quaternisés ou non, tels que les produits vendus sous la dénomination "GAFQUAT^{®} " par la société ISP comme par exemple "GAFQUAT^{®} 734" ou "GAFQUAT^{®} 755" ou bien les produits dénommés "COPOLYMER^{®} 845, 958 et 937". Ces polymères sont décrits en détail dans les brevets français 2.077.143 et 2.393.573,
- les terpolymères méthacrylate de diméthylaminoéthyle/vinylcaprolactame/vinylpyrrolidone tel que le produit commercialisé sous la dénomination GAFFIX VC 713 par la société ISP, et
- et les copolymères vinylpyrrolidone/méthacrylamide de diméthyl-amino-propyle quaternisés tels que les produits commercialisés sous la dénomination "GAFQUAT^{®} HS 100" par la société ISP.
   (2 les copolymères quaternaires de vinylpyrrolidone et de vinylimidazole ;
   (3) les chitosanes ou leurs sels; les sels utilisables sont en particulier les acétate, lactate, glutamate, gluconate ou le pyrrolidone-carboxylate de chitosane.

Parmi ces composés, on peut citer le chitosane ayant un taux de désacétylation de 90,5% en poids vendu sous la dénomination KYTAN BRUT STANDARD par la société ABER TECHNOLOGIES, le pyrrolidone-carboxylate de chitosane commercialisé sous la dénomination KYTAMER^{®} PC par la société AMERCHOL.

Les produits commercialisés répondant à cette définition sont plus particulièrement les produits vendus sous la dénomination "CELQUAT L 200" et "CELQUAT H 100" par la Société National Starch.

Le(s) polymère(s) fixant(s) amphotère(s) peu(ven)t être choisi(s) parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes ;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-a,β-dicarboxylique dont l'un des groupements carboxyliques a été amené à réagir avec une polyamine comportant un ou plusieurs groupements amines primaires ou secondaires.

Les polymères fixants amphotères répondant à la définition donnée ci-dessus plus particulièrement préférés sont choisis parmi les polymères suivants :
(1) les copolymères à motifs vinyliques acides et à motifs vinyliques basiques, tels que ceux résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique, tel que plus particulièrement les méthacrylate et acrylate de dialkyl-amino-alkyle, les dialkyl-amino-alkyl-méthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'atome d'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthyl-amino-éthyle avec le sulfate de diméthyle ou diéthyle.

Les acrylamides ou méthacrylamides N-substitués plus particulièrement préférés selon l'invention sont les composés dont les groupes alkyles comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.

Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.

Les comonomères basiques préférés sont des méthacrylates d'aminoéthyle, de butylaminoéthyle, de N,N'-diméthylaminoéthyle, de N-tertio-butylaminoéthyle.

On utilise particulièrement le copolymère dont la dénomination CTFA (4ème Ed., 1991) est Octylacrylamide/acrylates/butylaminoethyl-methacrylate copolymer, tel que les produits vendus sous la dénomination AMPHOMER^{®} ou LOVOCRYL^{®} 47 par la société NATIONAL STARCH.

(3) les polyaminoamides réticulés et acylés partiellement ou totalement dérivant de polyaminoamides de formule générale : 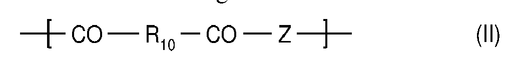 dans laquelle R10 représente un groupe divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :
a) dans les proportions de 60 à 100 % en moles, le groupe 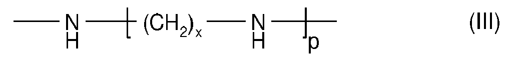
   où x=2 et p=2 ou 3, ou bien x=3 et p=2
   ce groupe dérivant de la diéthylène-triamine, de la triéthylène-tétraamine ou de la dipropylène-triamine;
b) dans les proportions de 0 à 40 % en moles, le groupe (III) ci-dessus, dans lequel x=2 et p=1 et qui dérive de l'éthylène-diamine, ou le groupe dérivant de la pipérazine : 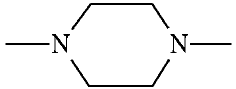
c) dans les proportions de 0 à 20 % en moles, le groupe -NH-(CH2)6-NH-dérivant de l'hexaméthylènediamine,
   ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et acylés par action d'acide acrylique, d'acide chloracétique ou d'une alcane-sultone ou de leurs sels.

Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.

Les alcane-sultones utilisées dans l'acylation sont de préférence la propane- ou la butane-sultone, les sels des agents d'acylation sont de préférence les sels de sodium ou de potassium.

(4) les polymères comportant des motifs zwittérioniques de formule : 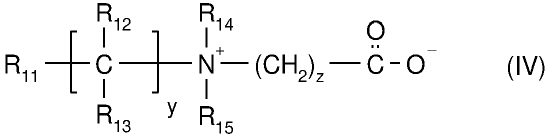 dans laquelle R11 désigne un groupement insaturé polymérisable tel qu'un groupement acrylate, méthacrylate, acrylamide ou méthacrylamide, y et z représentent un nombre entier de 1 à 3, R12 et R13 représentent un atome d'hydrogène, un groupe méthyle, éthyle ou propyle, R14 et R15 représentent un atome d'hydrogène ou un groupe alkyle de telle façon que la somme des atomes de carbone dans R14 et R15 ne dépasse pas 10.

Les polymères comprenant de tels motifs peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.

A titre d'exemple, on peut citer les copolymères méthacrylate de méthyle/diméthyl-carboxyméthylammonio-éthylméthacrylate de méthyle, tel que le produit vendu sous la dénomination DIAFORMER Z301 par la société SANDOZ.
(5) les polymères dérivés du chitosane comportant des motifs monomères répondant aux formules suivantes : 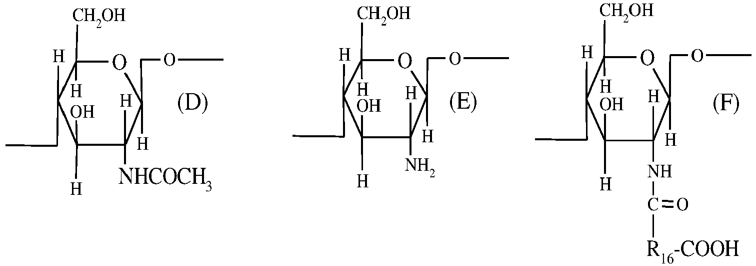
   le motif (D) étant présent dans des proportions comprises entre 0 et 30 %, le motif (E) dans des proportions comprises entre 5 et 50 % et le motif (F) dans des proportions comprises entre 30 et 90 %, étant entendu que dans ce motif (F), R16 représente un groupe de formule : 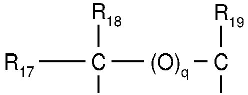
   dans laquelle si q=0, R17, R18 et R19, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R17, R18 et R19 étant dans ce cas un atome d'hydrogène ;
   ou si q=1, R17, R18 et R19 représentent chacun un atome d'hydrogène, ainsi que les sels formés par ces composés avec des bases ou des acides.
(6) Les polymères comprenant des motifs répondant à la formule générale (V) sont, par exemple, décrits dans le brevet français 1 400 366 : 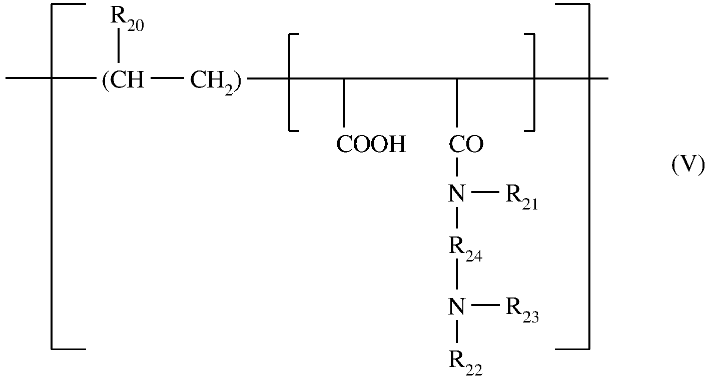 dans laquelle R20 représente un atome d'hydrogène, un groupe CH3O, CH3CH2O, phényle, R21 désigne un atome d'hydrogène ou un groupe alkyle inférieur tel que méthyle, éthyle, R22 désigne un atome d'hydrogène ou un groupe alkyle inférieur en C1-C6 tel que méthyle, éthyle, R23 désigne un groupe alkyle inférieur en C1-C6 tel que méthyle, éthyle ou un groupe répondant à la formule : -R24-N(R22)2, R24 représentant un groupement -CH2-CH2- , -CH2-CH2-CH2-, -CH2-CH(CH3)-, R22 ayant les significations mentionnées ci-dessus.
(7) Les polymères dérivés de la N-carboxyalkylation du chitosane comme le N-carboxyméthyl-chitosane ou le N-carboxybutyl-chitosane, vendus sous la dénomination « EVALSAN » par la société JAN DEKKER.
(8) Les polymères amphotères du type -D-X-D-X choisis parmi:
   a) les polymères obtenus par action de l'acide chloracétique ou le chloracétate de sodium sur les composés comportant au moins un motif de formule :

      -D-X-D-X-D- (VI)

      où D désigne un groupe 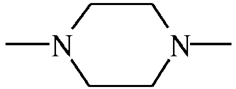
      et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      -D-X-D-X- (VI')

      où D désigne un groupe 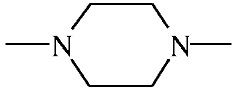
      et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyles et comportant un ou plusieurs atomes d'azote, l'atome d'azote étant substitué par une chaîne alkyle interrompue éventuellement par un atome d'oxygène et comportant obligatoirement une ou plusieurs fonctions carboxyles ou une ou plusieurs fonctions hydroxyles et bétaïnisées par réaction avec l'acide chloracétique ou du chloracétate de soude.
(9) les copolymères alkyl(C1-C5)vinyléther/anhydride maléique modifiés partiellement par semiamidification avec une N,N-dialkylaminoalkylamine telle que la N,N-diméthylaminopropylamine ou par semiestérification avec un N,N-dialkylaminoalcanol. Ces copolymères peuvent également comporter d'autres comonomères vinyliques tels que le vinylcaprolactame.

Parmi les polymères fixants amphotères cités ci-dessus, on citera ceux de la famille (3) tels que les copolymères dont la dénomination CTFA est Octylacrylamide/acrylates/butylamino-ethylmethacrylate copolymer, tels que les produits vendus sous les dénominations AMPHOMER^{®}, AMPHOMER^{®} LV 71 ou LOVOCRYL^{®} 47 par la société NATIONAL STARCH et ceux de la famille (4) tels que les copolymères méthacrylate de méthyle/diméthyl-carboxyméthylammonio-éthylméthacrylate de méthyle vendu par exemple sous la dénomination DIAFORMER Z301 par la société SANDOZ.

Le(s) polymère(s) fixant(s) non ionique(s) peu(ven)t être choisi(s), par exemple, parmi :
- les polyalkyloxazolines ;
- les homopolymères d'acétate de vinyle ;
- les copolymères d'acétate de vinyle tels que, par exemple, les copolymères d'acétate de vinyle et d'ester acrylique, les copolymères d'acétate de vinyle et d'éthylène, ou les copolymères d'acétate de vinyle et d'ester maléique, par exemple, de maléate de dibutyle ;
- les homopolymères et copolymères d'esters acryliques tels que, par exemple, les copolymères d'acrylates d'alkyle et de méthacrylates d'alkyle tels que les produits proposés par la société ROHM & HAAS sous les dénominations PRIMAL^{®} AC-261 K et EUDRAGIT^{®} NE 30 D, par la société BASF sous la dénomination 8845, par la société HOECHST sous la dénomination APPRETAN^{®} N9212 ;
- les copolymères d'acrylonitrile et d'un monomère non ionique choisis, par exemple, parmi le butadiène et les (méth)acrylates d'alkyle ; on peut citer les produits proposés sous la dénomination CJ 0601 B par la société ROHM & HAAS ;
- les homopolymères de styrène ;
- les copolymères de styrène comme, par exemple, les copolymères de styrène et de (méth)acrylate d'alkyle tels que les produits MOWILITH^{®} LDM 6911, MOWILITH^{®} DM 611 et MOWILITH^{®} LDM 6070 proposés par la société HOECHST, les produits RHODOPAS^{®} SD 215 et RHODOPAS^{®} DS 910 proposés par la société RHONE POULENC ; les copolymères de styrène, de méthacrylate d'alkyle et d'acrylate d'alkyle ; les copolymères de styrène et de butadiène ; ou les copolymères de styrène, de butadiène et de vinylpyridine ;
- les polyamides ;
- les homopolymères de vinyllactame, tels que les homo-polymères de vinylpyrrolidone, le polyvinylcaprolactame commercialisé sous la dénomination Luviskol^{®} PLUS par la société BASF ; et
- les copolymères de vinyllactame tels qu'un copolymère poly(vinylpyrrolidone/vinyllactame) vendu sous le nom commercial Luvitec^{®} VPC 55K65W par la société BASF, les copolymères poly(vinylpyrrolidone/acétate de vinyle) comme ceux commercialisés sous la dénomination PVPVA^{®} S630L par la société ISP, Luviskol^{®} VA 73, VA 64, VA 55, VA 37 et VA 28 par la société BASF ; et les terpolymères poly(vinylpyrrolidone/acétate de vinyle/propionate de vinyle) comme, par exemple, celui commercialisé sous la dénomination Luviskol^{®} VAP 343 par la société BASF.

Les groupes alkyles des polymères non ioniques mentionnés ci-dessus ont, de préférence, de 1 à 6 atomes de carbone.

On peut également utiliser à l'étape a) comme polymères fixants, des polyuréthanes fonctionnalisés ou non, cationiques, non-ioniques, anioniques ou amphotères, ou leurs mélanges.

Les polyuréthanes particulièrement visés par la présente invention sont ceux décrits dans les demandes EP 0 751 162, EP 0 637 600, EP 0 648 485 et FR 2 743 297 dont la demanderesse est titulaire, ainsi que dans les demandes EP 0 656 021 et WO 94/03510 de la société BASF, et EP 0 619 111 de la société National Starch.

Comme polyuréthanes convenant particulièrement bien dans la présente invention, on peut citer les produits commercialisés sous la dénomination LUVISET PUR^{®} par la société BASF.

Par polymère conditionneur on entend au sens de la présente invention un polymère capable d'apporter aux fibres kératiniques et en particulier aux cheveux une amélioration d'au moins une des propriétés suivantes : douceur au toucher, effet de lissage, aptitude au démêlage.

De préférence le(s) polymère(s) conditionneur(s) est(sont) un(des) polymère(s) cationique(s) ou un(des) polymère(s) amphotère(s), notamment le Polyquaternium-22.

De préférence le(s) polymère(s) conditionneur(s) est(sont) choisi(s) parmi :
(1) les cyclopolymères de sels d'alkyl diallyl amine ou de dialkyl diallyl ammonium tels que les homopolymères ou copolymères comportant comme constituant principal de la chaîne des motifs répondant aux formules (VII) ou (VIII) : 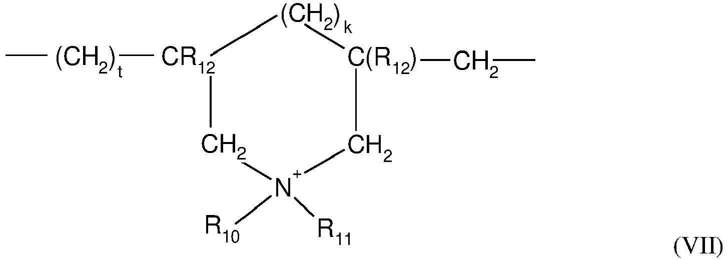 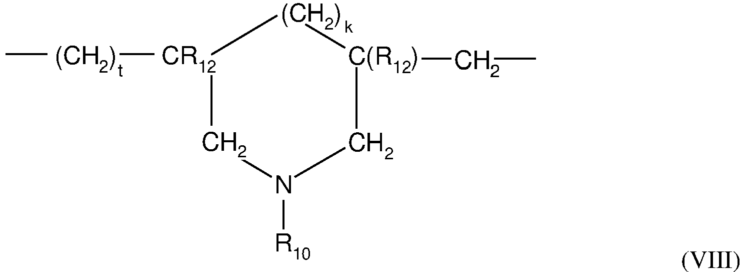 dans lesquelles k et t sont égaux à 0 ou 1, la somme k + t étant égale à 1, R12 désigne un atome d'hydrogène ou un groupe méthyle, R10 et R11, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C1-C4), ou R10 et R11 peuvent désigner conjointement avec l'atome d'azote auquel ils sont rattachés, des groupement hétérocycliques, tels que pipéridinyle ou morpholinyle, Y- est un anion tel que bromure, chlorure, acétate, borate, citrate, tartrate, bisulfate, bisulfite, sulfate, phosphate. Ces polymères sont notamment décrits dans le brevet français 2 080 759 et dans son certificat d'addition 2 190 406.

R10 et R11, indépendamment l'un de l'autre, désignent de préférence un groupement alkyle ayant de 1 à 4 atomes de carbone.

Parmi les polymères définis ci-dessus, on peut citer plus particulièrement l'homopolymère de chlorure de diméthyldiallylammonium vendu sous la dénomination "Merquat 100" par la société NALCO, ses homologues de faibles masses moléculaires moyenne en poids, et les copolymères de chlorure de diallyldiméthylammonium et d'acrylamide commercialisés sous la dénomination "MERQUAT 550".

(2) les polymères de diammonium quaternaire contenant notamment des motifs récurrents répondant à la formule (IX) : 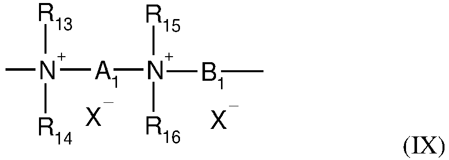 dans laquelle :
R13, R14, R15 et R16, identiques ou différents, représentent des groupes aliphatiques, alicycliques ou arylaliphatiques contenant de 1 à 20 atomes de carbone ou des radicaux hydroxyalkylaliphatiques inférieurs, ou bien R13, R14, R15 et R16, ensemble ou séparément, constituent avec les atomes d'azote auxquels ils sont rattachés des hétérocycles contenant éventuellement un second hétéroatome autre que l'azote, ou bien R13, R14, R15 et R16 représentent un groupe alkyle en C1-C6, linéaire ou ramifié, substitué par un groupement nitrile, ester, acyle, amide ou -CO-O-R17-D ou -CO-NH-R17-D où R17 est un alkylène et D un groupement ammonium quaternaire,
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X- désigne un anion dérivé d'un acide minéral ou organique,
A1, R13 et R15 peuvent former avec les deux atomes d'azote auxquels ils sont rattachés un cycle pipérazinique. En outre, si A1 désigne un radical alkylène ou hydroxyalkylène, linéaire ou ramifié, saturé ou insaturé, B1 peut également désigner un groupement (CH2)n-CO-D-OC-(CH2)p-,
n et p sont des nombres entiers variant de 2 à 20 environ,
dans lequel D désigne :
   a) un reste de glycol de formule : -O-Z-O-, où Z désigne un radical hydrocarboné, linéaire ou ramifié, ou un groupement répondant à l'une des formules suivantes :

      -(CH2-CH2-O)x -CH2-CH2-,

      -[CH2-CH(CH3)-O]y-CH2-CH(CH3)-,

      où x et y désignent un nombre entier de 1 à 4, représentant un degré de polymérisation défini et unique ou un nombre quelconque de 1 à 4 représentant un degré de polymérisation moyen,
   b) un reste de diamine bis-secondaire tel qu'un dérivé de pipérazine,
   c) un reste de diamine bis-primaire de formule : -NH-Y-NH-, où Y désigne un radical hydrocarboné linéaire ou ramifié, ou bien le radical bivalent -CH2-CH2-S-S-CH2-CH2-,
   d) un groupement uréylène de formule -NH-CO-NH-.

De préférence, X- est un anion tel que le chlorure ou le bromure.

Ces polymères ont une masse moléculaire moyenne en nombre généralement comprise entre 1 000 et 100 000.

Des polymères de ce type sont notamment décrits dans les brevets français 2 320 330, 2 270 846, 2 316 271, 2 336 434 et 2 413 907 et les brevets US 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 et 4 027 020.

On peut utiliser plus particulièrement les polymères qui sont constitués de motifs récurrents répondant à la formule (X) : 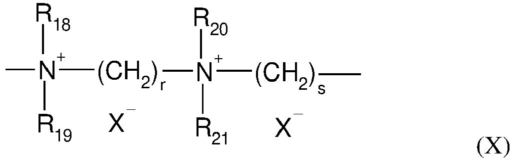 dans laquelle R18, R19, R20 et R21, identiques ou différents, désignent un groupe alkyle ou hydroxyalkyle ayant de 1 à 4 atomes de carbone environ, r et s sont des nombres entiers variant de 2 à 20 environ et, X- est un anion dérivé d'un acide minéral ou organique.

Un composé de formule (X) particulièrement préféré est celui pour lequel R18, R19, R20 et R21 représentent un radical méthyle, r = 3, s = 6 et X = Cl, dénommé Hexadimethrine chloride selon la nomenclature INCI (CTFA).

Les polymères de diammonium quaternaires peuvent être aussi constitués notamment de motifs de formule (XI) : 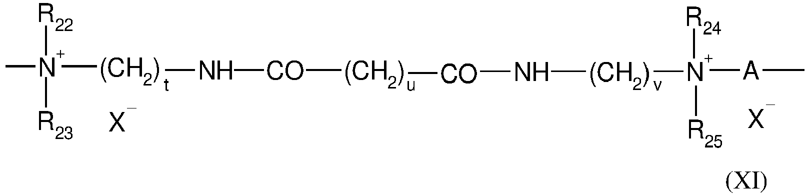 dans laquelle :
R22, R23, R24 et R25, identiques ou différents, représentent un atome d'hydrogène ou un groupe méthyle, éthyle, propyle, β-hydroxyéthyle, β-hydroxypropyle ou -CH2CH2(OCH2CH2)pOH,
où p est égal à 0 ou à un nombre entier compris entre 1 et 6, sous réserve que R22, R23, R24 et R25 ne représentent pas simultanément un atome d'hydrogène,
t et u, identiques ou différents, sont des nombres entiers compris entre 1 et 6,
v est égal à 0 ou à un nombre entier compris entre 1 et 34,
X- désigne un anion tel qu'un halogènure,
A désigne un radical d'un dihalogénure ou représente de préférence -CH2-CH2-O-CH2-CH2-.

De tels composés sont notamment décrits dans la demande de brevet EP-A-122 324.

On peut, par exemple, citer parmi ceux-ci, les produits "Mirapol^{®} A 15", "Mirapol^{®} AD1", "Mirapol^{®} AZ1" et "Mirapol^{®} 175" vendus par la société MIRANOL.

(3) les polysaccharides cationiques, notamment les celluloses et les gommes de galactomannane cationiques.

Pour les celluloses on peut citer les copolymères de cellulose ou de dérivés de cellulose greffés avec un monomère hydrosoluble comportant un ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyloxyéthyl triméthylammonium, de méthacrylamidopropyl triméthylammonium, de diméthyl-diallylammonium.

Les produits commercialisés répondant à cette définition sont plus particulièrement les produits vendus sous la dénomination "CELQUAT L 200", « CELQUAT LOR » et "CELQUAT H 100" par la Société AKZO NOBEL (Polyquaternium-4). On peut également citer le produit vendu sous la dénomination MERQUAT 280 (Polyquaternium-22) par la société LUBRIZOL.

### Tensioactifs

La composition de l'étape a) est une composition contenant au moins un tensioactif, ladite composition étant exempte d'hydroxydes alcalins ou alcalino-terreux à un pH supérieur à 12 ou d'agents réducteurs permettant la rupture des liaisons disulfures.

La composition comprend de préférence de 30 à 70 % en poids d'eau par rapport au poids total de la composition.

De préférence ladite composition comprend au moins un tensioactif dans une teneur variant de 0,01 % à 95 %, de préférence, de 0,1 % à 60 %, mieux de 1 à 50 %, en poids par rapport au poids total de la composition.

Au sens de l'invention, le ou les tensioactifs utilisés à l'étape a) peu(ven)t être choisi(s) parmi les tensioactifs non ioniques, les tensioactifs anioniques, les tensioactifs cationiques et les tensioactifs amphotères ou zwiterrioniques.

Le ou les tensioactifs conformes à l'invention peu(ven)t être siliconé(s) ou non.

On entend par «agent tensioactif anionique», un tensioactif ne comportant à titre de groupements ioniques ou ionisables que des groupements anioniques. Ces groupements anioniques sont choisis de préférence parmi les groupements -C(O)OH, -C(O)O-, -SO₃H, -S(O)₂O-, -OS(O)₂OH, -OS(O)₂O-, -P(O)OH₂, -P(O)₂O-, -P(O)O₂-, -P(OH)₂, =P(O)OH, -P(OH)O-, =P(O)O-, =POH, =PO-, les parties anioniques comprenant un contre ion cationique tel qu'un métal alcalin, un métal alcalino-terreux, ou un ammonium.

A titre d'exemples d'agents tensioactifs anioniques utilisables dans l'étape a) selon l'invention, on peut citer les alkyl sulfates, les alkyl éther sulfates, les alkylamidoéthersulfates, les alkylarylpolyéthersulfates, les monoglycéride-sulfates, les alkylsulfonates, les alkylamidesulfonates, les alkylarylsulfonates, les alpha-oléfine-sulfonates, les paraffine-sulfonates, les alkylsulfosuccinates, les alkyléthersulfo-succinates, les alkylamide-sulfosuccinates, les alkylsulfo-acétates, les acylsarcosinates, les acylglutamates, les alkylsulfosuccinamates, les acyliséthionates et les N-acyltaurates, les sels de monoesters d'alkyle et d'acides polyglycoside-polycarboxyliques, les acyllactylates, les sels d'acides D-galactoside-uroniques, les sels d'acides alkyl éther-carboxyliques, les sels d'acides alkyl aryl éther-carboxyliques, les sels d'acides alkyl amidoéther-carboxyliques, et les formes non salifiées correspondantes de tous ces composés, les groupes alkyle et acyle de tous ces composés comportant de 6 à 40 atomes de carbone et le groupe aryle désignant un groupe phényle.

Ces composés peuvent être oxyéthylénés et comportent alors de préférence de 1 à 50 motifs oxyde d'éthylène.

Les sels de monoesters d'alkyle en C₆-C₂₄ et d'acides polyglycoside-polycarboxyliques peuvent être choisis parmi les polyglycoside-citrates d'alkyle en C₆-C₂₄, les polyglycosides-tartrates d'alkyle en C₆-C₂₄ et les polyglycoside-sulfosuccinates d'alkyle en C₆-C₂₄.

Lorsque l'agent ou les agents tensioactifs anioniques est (sont) sous forme de sel, il(s) peu(ven)t être choisi(s) parmi les sels de métaux alcalins tels que le sel de sodium ou de potassium et de préférence de sodium, les sels d'ammonium, les sels d'amines et en particulier d'aminoalcools ou les sels de métaux alcalino-terreux tel que les sels de magnésium.

A titre d'exemple de sels d'aminoalcools, on peut citer notamment les sels de mono-, di- et triéthanolamine, les sels de mono-, di- ou triisopropanolamine, les sels de 2-amino-2-méthyl-1-propanol, 2-amino-2-méthyl-1,3-propanediol et tris(hydroxyméthyl)amino méthane.

On utilise de préférence les sels de métaux alcalins ou alcalino-terreux et en particulier les sels de sodium ou de magnésium.

Parmi les agents tensioactifs anioniques cités, on préfère utiliser les alkyl(C₆-C₂₄)sulfates, les alkyl(C₆-C₂₄)éthersulfates comprenant de 2 à 50 motifs oxyde d'éthylène, notamment sous forme de sels de métaux alcalins, d'ammonium, d'aminoalcools, et de métaux alcalino-terreux, ou un mélange de ces composés.

En particulier, on préfère utiliser les alkyl(C₁₂-C₂₀)sulfates, les alkyl(C₁₂-C₂₀)éthersulfates comprenant de 2 à 20 motifs oxyde d'éthylène, notamment sous forme de sels de métaux alcalins, d'ammonium, d'aminoalcools, et de métaux alcalino-terreux, ou un mélange de ces composés. Mieux encore, on préfère utiliser le lauryl éther sulfate de sodium à 2,2 moles d'oxyde d'éthylène.

Des exemples d'agents tensioactifs non-ioniques utilisables dans l'étape a) selon l'invention sont décrits par exemple dans "Handbook of Surfactants" par M.R. PORTER, éditions Blackie & Son (Glasgow and London), 1991, pp 116-178. Ils sont choisis notamment parmi les alcools, les alpha-diols, les alkyl(C₁-C₂₀)phénols, ces composés étant polyéthoxylés, polypropoxylés et/ou polyglycérolés, et ayant au moins une chaîne grasse comportant, par exemple, de 8 à 40 atomes de carbone, le nombre de groupements oxyde d'éthylène et/ou oxyde de propylène pouvant aller notamment de 1 à 200 et le nombre de groupements glycérol pouvant aller notamment de 1 à 30.

On peut également citer les copolymères d'oxyde d'éthylène et de propylène, les esters d'acides gras et de sorbitan éventuellement oxyéthylénés, les esters d'acides gras et de saccharose, les esters d'acides gras polyoxyalkylénés, les alkylpolyglycosides éventuellement oxyalkylénés, les esters d'alkylglucosides, les mono ou di alcanolamides, les dérivés de N-alkylglucamine et de N-acyl-méthylglucamine, les aldobionamides, les silicones oxyéthylénées et/ou oxypropylénées et les oxydes d'amine

Le ou les tensioactifs non ioniques utilisé(s) à l'étape a) est (sont) plus particulièrement choisi(s) parmi les tensioactifs non ioniques mono ou poly- oxyalkylénés, mono- ou poly- glycérolés. Les motifs oxyalkylénés sont plus particulièrement des motifs oxyéthylénés, oxypropylénés, ou leur combinaison, de préférence oxyéthylénés.

A titre d'exemples de tensioactifs non ioniques oxyalkylénés, on peut citer :
- les alkyl(C₈-C₂₄)phénols oxyalkylénés,
- les alcools en C₈-C₃₀, saturés ou non, linéaires ou ramifiés, oxyalkylénés, comme notamment le déceth-3 ou déceth-5,
- les amides, en C₈-C₃₀, saturés ou non, linéaires ou ramifiés, oxyalkylénés,
- les esters d'acides en C₈-C₃₀, saturés ou non, linéaires ou ramifiés, et de polyéthylèneglycols,
- les esters d'acides en C₈-C₃₀, saturés ou non, linéaires ou ramifiés, et de sorbitol polyoxyéthylénés,
- les huiles végétales oxyéthylénées, saturées ou non,
- les condensats d'oxyde d'éthylène et/ou d'oxyde de propylène, entre autres, seuls ou en mélanges,
- les silicones oxyéthylénées et/ou oxypropylénées.

Le(s) tensioactif(s) présente(nt) un nombre de moles d'oxyde d'éthylène et/ou de propylène allant de préférence de 1 à 100, mieux de 2 à 50. De manière avantageuse, le(s) tensioactif(s) non ioniques ne comprend(comprennent) pas de motifs oxypropylénés.

Conformément à un mode de réalisation préféré de l'invention, le(s) tensioactif(s) non ioniques utilisés à l'étape a) est(sont) choisi(s) parmi les alcools en C₈-C₃₀, oxyéthylénés, les esters d'acides en C₈-C₃₀, saturés ou non, linéaires ou ramifiés, et de sorbitol polyoxyéthylénés.

A titre d'exemple de tensioactifs non ioniques mono- ou poly- glycérolés, on utilise à l'étape a) de préférence les alcools en C₈-C₄₀, mono- ou poly- glycérolés.

En particulier, les alcools en C₈-C₄₀ mono- ou poly- glycérolés correspondent à la formule (A1) suivante :

RO-[CH₂-CH(CH₂OH)-O]ₘ-H (A1)

dans laquelle R représente un radical alkyle ou alcényle, linéaire ou ramifié, en C₈-C₄₀, de préférence en C₈-C₃₀, et m représente un nombre allant de 1 à 30 et de préférence de 1 à 10.

A titre d'exemple de composés utilisables à l'étape a) dans le cadre de l'invention, on peut citer, l'alcool laurique à 4 moles de glycérol (nom INCI : POLYGLYCERYL-4 LAURYL ETHER), l'alcool laurique à 1,5 moles de glycérol, l'alcool oléique à 4 moles de glycérol (nom INCI : POLYGLYCERYL-4 OLEYL ETHER), l'alcool oléique à 2 moles de glycérol (Nom INCI : POLYGLYCERYL-2 OLEYL ETHER), l'alcool cétéarylique à 2 moles de glycérol, l'alcool cétéarylique à 6 moles de glycérol, l'alcool oléocétylique à 6 moles de glycérol, et l'octadécanol à 6 moles de glycérol.

L'alcool peut représenter un mélange d'alcools au même titre que la valeur de m représente une valeur statistique, ce qui signifie que dans un produit commercial peuvent coexister plusieurs espèces d'alcools gras polyglycérolés sous forme d'un mélange.

Parmi les alcools mono- ou poly-glycérolés, on préfère plus particulièrement utiliser l'alcool en C₈/C₁₀ à une mole de glycérol, l'alcool en C₁₀/C₁₂ à 1 mole de glycérol et l'alcool en C₁₂ à 1,5 mole de glycérol.

De préférence le(s) tensioactif(s) non ionique(s) est(sont) choisi(s) parmi les alcanolamides oxyéthylénés ou non et les alcools gras oxyéthylénés.

Le ou les agents tensioactif(s) cationique(s) utilisable(s) à l'étape a) comprend(comprennent) par exemple les sels d'amines grasses primaire, secondaire ou tertiaire, éventuellement polyoxyalkylénées, les sels d'ammonium quaternaire, et leurs mélanges.

A titre de sels d'ammonium quaternaire, on peut notamment citer, par exemple:
- ceux répondant à la formule générale (A2) suivante :

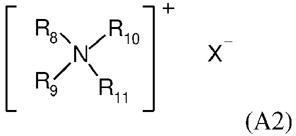

Formule (A2) dans laquelle :
R₈ à Ru, identiques ou différents, représentent un groupe aliphatique, linéaire ou ramifié, comportant de 1 à 30 atomes de carbone, ou un groupe aromatique tel que aryle ou alkylaryle, étant entendu qu'au moins un des groupes R₈ à Ru comportent de 8 à 30 atomes de carbone, et de préférence de 12 à 24 atomes de carbone ; et
X⁻ représente un contre ion anionique organique ou inorganique, tel que celui choisi parmi les halogénures, acétates, phosphates, nitrates, alkyl(C₁-C₄)sulfates, alkyl(C₁-C₄)- ou alkyl(C₁-C₄)aryl-sulfonates, en particulier méthylsulfate et éthylsulfate.

Les groupes aliphatiques de R₈ à Ru peuvent en outre comporter des hétéroatomes tels que notamment l'oxygène, l'azote, le soufre et les halogènes.

Les groupes aliphatiques de R₈ à Ru sont par exemple choisis parmi les groupes alkyle en C₁-C₃₀ ou alkényle, alcoxy en C₁-C₃₀, polyoxyalkylène (C₂-C₆), alkylamide en C₁-C₃₀, alkyl(C₁₂-C₂₂)amidoalkyle(C₂-C₆), alkyl(C₁₂-C₂₂)acétate, et hydroxyalkyle en C₁-C₃₀, X⁻ est un contre ion anionique choisi parmi les halogénures, phosphates, acétates, lactates, alkyl(C₁-C₄)sulfates, alkyl(C₁-C₄)- ou alkyl(C₁-C₄)aryl-sulfonates.

Parmi les sels d'ammonium quaternaire de formule (A2), on préfère d'une part, les chlorures de tétraalkylammonium comme, par exemple, les chlorures de dialkyldiméthylammonium ou d'alkyltriméthylammonium dans lesquels le groupe alkyle comporte environ de 12 à 22 atomes de carbone, en particulier les chlorures de béhényl triméthylammonium, de distéaryldiméthyl-ammonium, de cétyltriméthylammonium, de benzyldiméthylstéarylammonium ou encore, d'autre part, le méthosulfate de distéaroyléthylhydroxyéthylméthylammonium, le méthosulfate de dipalmitoyléthylhydroxy éthylammonium ou le méthosulfate de distéaroyléthylhydroxyéthylammonium, ou encore, enfin, le chlorure de palmitylamidopropyltriméthylammonium ou le chlorure de stéaramidopropyldiméthyl-(myristyl acétate)-ammonium commercialisé sous la dénomination CERAPHYL^{®} 70 par la société VAN DYK ;
- les sels d'ammonium quaternaire de l'imidazoline, comme par exemple ceux de formule (A3) suivante :

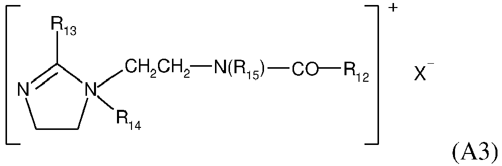

Formule (A3) dans laquelle :
R₁₂ représente un groupe alcényle ou alkyle comportant de 8 à 30 atomes de carbone, par exemple dérivés des acides gras du suif ;
R₁₃ représente un atome d'hydrogène, un groupe alkyle en C₁-C₄ ou un groupe alcényle ou alkyle comportant de 8 à 30 atomes de carbone ;
R₁₄ représente un groupe alkyle en C₁-C₄ ;
R₁₅ représente un atome d'hydrogène, un groupe alkyle en C₁-C₄ ;
X- représente un contre ion anionique organique ou inorganique, tel que celui choisi parmi les halogénures, phosphates, acétates, lactates, alkyl(C₁-C₄)sulfates, alkyl(C₁-C₄)- ou alkyl(C₁-C₄)aryl-sulfonates.

De préférence, R₁₂ et R₁₃ désignent un mélange de groupes alcényle ou alkyle comportant de 12 à 21 atomes de carbone, par exemple dérivés des acides gras du suif, R₁₄ désigne un groupe méthyle, R₁₅ désigne un atome d'hydrogène. Un tel produit est par exemple commercialisé sous la dénomination REWOQUAT^{®} W 75 par la société REWO ;
- les sels de di- ou de triammonium quaternaire en particulier de formule (A4) suivante :

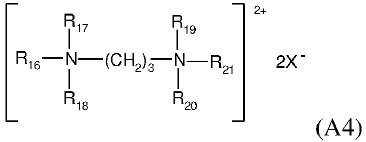

Formule (A4) dans laquelle :
R₁₆ désigne un groupe alkyle comportant environ de 16 à 30 atomes de carbone, éventuellement hydroxylé et/ou interrompu par un ou plusieurs atomes d'oxygène ;
R₁₇ est choisi parmi l'hydrogène, un groupe alkyle comportant de 1 à 4 atomes de carbone ou un groupe -(CH₂)₃-N⁺(R₁₆ₐ)(R₁₇ₐ)(R₁₈ₐ), X⁻ ;
R₁₆ₐ, R₁₇ₐ, R₁₈ₐ, R₁₈, R₁₉, R₂0 et R₂₁, identiques ou différents, sont choisis parmi l'hydrogène et un groupe alkyle comportant de 1 à 4 atomes de carbone ; et
X⁻, identiques ou différents, représentent un contre ion anionique organique ou inorganique, tels que celui choisi parmi les halogénures, acétates, phosphates, nitrates, alkyl(C₁-C₄)sulfates, alkyl(C₁-C₄)- ou alkyl(C₁-C₄)aryl-sulfonates, en particulier méthylsulfate et éthylsulfate.

De tels composés sont par exemple le Finquat CT-P proposé par la société FINETEX (Quaternium 89), le Finquat CT proposé par la société FINETEX (Quaternium 75) ;
- les sels d'ammonium quaternaire contenant une ou plusieurs fonctions esters, tels que ceux de formule (A5) suivante :

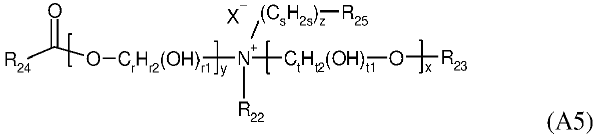

Formule (A5) dans laquelle :
R₂₂ est choisi parmi les groupes alkyles en C₁-C₆ et les groupes hydroxyalkyle ou dihydroxyalkyle en C₁-C₆,
R₂₃ est choisi parmi :
   - le groupe 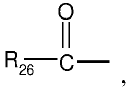
   - les groupes R₂₇ hydrocarbonés en C₁-C₂₂, linéaires ou ramifiés, saturés ou insaturés,
   - l'atome d'hydrogène,
R₂₅ est choisi parmi :
   - le groupe 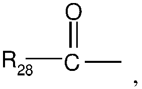
   - les groupes R₂₉ hydrocarbonés en C₁-C₆, linéaires ou ramifiés, saturés ou insaturés,
   - l'atome d'hydrogène,
R₂₄, R₂₆ et R₂₈, identiques ou différents, sont choisis parmi les groupes hydrocarbonés en C₇-C₂₁, linéaires ou ramifiés, saturés ou insaturés ;
r, s et t, identiques ou différents, sont des entiers valant de 2 à 6,
r1 et t1 identiques ou différents, valant 0 ou 1 avec r2+r1=2r et t1+t2=2t
y est un entier valant de 1 à 10,
x et z, identiques ou différents, sont des entiers valant de 0 à 10,
X- représente un contre ion anionique, organique ou inorganique,
sous réserve que la somme x + y + z vaut de 1 à 15, que lorsque x vaut 0 alors R₂₃ désigne R₂₇ et que lorsque z vaut 0 alors R₂₅ désigne R₂₉.

Les groupes alkyles R₂₂ peuvent être linéaires ou ramifiés, et plus particulièrement linéaires.

De préférence, R₂₂ désigne un groupe méthyle, éthyle, hydroxyéthyle ou dihydroxypropyle, et plus particulièrement un groupe méthyle ou éthyle.

Avantageusement, la somme x + y + z vaut de 1 à 10.

Lorsque R₂₃ est un groupe R₂₇ hydrocarboné, il peut être long et avoir de 12 à 22 atomes de carbone, ou court et avoir de 1 à 3 atomes de carbone.

Lorsque R₂₅ est un groupe R₂₉ hydrocarboné, il a de préférence 1 à 3 atomes de carbone.

Avantageusement, R₂₄, R₂₆ et R₂₈, identiques ou différents, sont choisis parmi les groupes hydrocarbonés en C₁₁-C₂₁, linéaires ou ramifiés, saturés ou insaturés, et plus particulièrement parmi les groupes alkyle et alcényle en C₁₁-C₂₁, linéaires ou ramifiés, saturés ou insaturés.

De préférence, x et z, identiques ou différents, valent 0 ou 1.

Avantageusement, y est égal à 1.

De préférence, r, s et t, identiques ou différents, valent 2 ou 3, et encore plus particulièrement sont égaux à 2.

Le contre ion anionique X- est de préférence un halogénure, tel que chlorure, bromure ou iodure ; un alkyl(C₁-C₄)sulfate ; alkyl(C₁-C₄)- ou alkyl(C₁-C₄)aryl-sulfonate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

Le contre ion anionique X⁻ est encore plus particulièrement le chlorure, le méthylsulfate ou l'éthylsulfate.

On utilise plus particulièrement dans la composition selon l'invention, les sels d'ammonium de formule (A5) dans laquelle :
- R₂₂ désigne un groupe méthyle ou éthyle,
- x et y sont égaux à 1,
- z est égal à 0 ou 1,
- r, s et t sont égaux à 2,
- R₂₃ est choisi parmi :
   le groupe 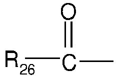
   les groupes méthyle, éthyle ou hydrocarbonés en C₁₄-C₂₂,
   l'atome d'hydrogène,
- R₂₅ est choisi parmi :
   le groupe 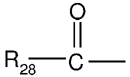
   l'atome d'hydrogène,
- R₂₄, R₂₆ et R₂₈, identiques ou différents, sont choisis parmi les groupes hydrocarbonés en C₁₃-C₁₇, linéaires ou ramifiés, saturés ou insaturés, et de préférence parmi les groupes alkyle et alcényle en C₁₃-C₁₇, linéaires ou ramifiés, saturés ou insaturés.

Avantageusement, les radicaux hydrocarbonés sont linéaires.

On peut citer par exemple parmi les composés de formule (A5) les sels, notamment le chlorure ou le méthylsulfate, de diacyloxyéthyldiméthylammonium, de diacyloxyéthylhydroxyéthylméthylammonium, de monoacyloxyéthyldihydroxyéthyl méthylammonium, de triacyloxyéthylméthylammonium, de monoacyloxy éthylhydroxy éthyldiméthylammonium, et leurs mélanges. Les groupes acyles ont de préférence 14 à 18 atomes de carbone et proviennent plus particulièrement d'une huile végétale comme l'huile de palme ou de tournesol. Lorsque le composé contient plusieurs groupes acyles, ces derniers peuvent être identiques ou différents.

Ces produits sont obtenus, par exemple, par estérification directe de la triéthanolamine, de la triisopropanolamine, d'alkyldiéthanolamine ou d'alkyldiiso propanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle, de préférence de méthyle ou d'éthyle, un sulfate de dialkyle, de préférence de méthyle ou d'éthyle, le méthanesulfonate de méthyle, le para-toluènesulfonate de méthyle, la chlorhydrine du glycol ou du glycérol.

De tels composés sont par exemple commercialisés sous les dénominations DEHYQUART^{®} par la société HENKEL, STEPANQUAT^{®} par la société STEPAN, NOXAMIUM^{®} par la société CECA, REWOQUAT^{®} WE 18 par la société REWO-WITCO.

La composition selon l'invention peut contenir, par exemple, un mélange de sels de mono-, di- et triester d'ammonium quaternaire avec une majorité en poids de sels de diester.

On peut aussi utiliser les sels d'ammonium contenant au moins une fonction ester décrits dans les brevets US-A-4874554 et US-A-4137180.

On peut utiliser le chlorure de behenoylhydroxypropyltriméthylammonium proposé par KAO sous la dénomination Quatarmin BTC 131.

De préférence les sels d'ammonium contenant au moins une fonction ester contiennent deux fonctions esters.

Parmi les agents tensioactifs cationiques pouvant être présents dans la composition selon l'invention, on préfère plus particulièrement choisir les sels de cétyltriméthylammonium, de béhényltriméthylammonium, de dipalmitoyléthyl-hydroxyéthylméthylammonium, et leurs mélanges, et plus particulièrement le chlorure de béhényltriméthylammonium, le chlorure de cétyltriméthylammonium, le méthosulfate de dipalmitoyléthylhydroxyéthylammonium, et leurs mélanges.

Le ou les agents tensioactif(s) amphotère(s) ou zwittérionique(s) utilisé(s) à l'étape a) est(sont) de préférence non siliconé(s). Il(s) peu(ven)t être notamment des dérivés d'amines aliphatiques secondaires ou tertiaires, dans lesquels le groupe aliphatique est une chaîne linéaire ou ramifiée comportant 8 à 22 atomes de carbone et contenant, au moins un groupe anionique hydrosolubilisant tel que, par exemple, un groupe carboxylate, sulfonate, sulfate, phosphate ou phosphonate, on peut citer encore les alkyl(C₈-C₂₀)bétaïnes, les sulfobétaïnes, les alkyl(C₈-C₂₀)amidoalkyl(C₆-C₈)-bétaïnes ou les alkyl(C₈-C₂₀)amidoalkyl(C₆-_{C8})sulfobétaïnes ; et leurs mélanges.

Parmi les dérivés d'amines aliphatiques secondaires ou tertiaires, éventuellement quaternisées utilisables, tels que définis ci-dessus, on peut également citer les composés de structures respectives (A6) et (A7) suivantes :

Rₐ-C(O)-NH-CH₂-CH₂-N⁺(R_{b})(R_{c})-CH₂C(O)O⁻, M⁺ , X⁻ (A6)

Formule (A6) dans laquelle :
▪ Rₐ représente un groupe alkyle ou alcényle en C₁₀-C₃₀ dérivé d'un acide RₐCOOH, de préférence présent dans l'huile de coprah hydrolysée, un groupe heptyle, nonyle ou undécyle ;
▪ R_{b} représente un groupe bêta-hydroxyéthyle ; et
▪ R_{c} représente un groupe carboxyméthyle ;
▪ M⁺ représente un contre ion cationique issu d'un métal alcalin, alcalinoterreux, tel que le sodium, un ion ammonium ou un ion issu d'une amine organique, et
▪ X⁻ représente un contre ion anionique organique ou inorganique, tel que celui choisi parmi les halogénures, acétates, phosphates, nitrates, alkyl(Ci-C₄)sulfates, alkyl(C₁-C₄)- ou alkyl(C₁-C₄)aryl-sulfonates, en particulier méthylsulfate et éthylsulfate ; ou alors M⁺ et X⁻ sont absents ;

   R_{a'}-C(O)-NH-CH2-CH2-N(B)(B') (A7)

Formule (A7) dans laquelle :
▪ B représente le groupe -CH₂-CH₂-O-X' ;
▪ B' représente le groupe -(CH2)_{z}Y', avec z = 1 ou 2 ;
▪ X' représente le groupe -CH₂-C(O)OH, -CH₂-C(O)OZ', -CH₂-CH₂-C(O)OH, -CH₂-CH₂-C(O)OZ', ou un atome d'hydrogène ;
▪ Y' représente le groupe -C(O)OH, -C(O)OZ', -CH₂-CH(OH)-SO₃H ou le groupe -CH₂-CH(OH)-SO₃-Z' ;
▪ Z' représente un contre ion cationique issu d'un métal alcalin ou alcalinoterreux, tel que le sodium, un ion ammonium ou un ion issu d'une amine organique ;
▪ R_{a'} représente un groupe alkyle ou alcényle en C₁₀-C₃₀ d'un acide R_{a'}-C(O)OH de préférence présent dans l'huile de coprah ou dans l'huile de lin hydrolysée, un groupe alkyle, notamment en C₁₇ et sa forme iso, un groupe en C₁₇ insaturé.

Ces composés de formule (A6) et (A7) sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL^{®} C2M concentré.

On peut aussi utiliser des composés de formule (A'2) ;

Ra"-NH-CH(Y")-(CH₂)ₙ-C(O)-NH-(CH₂)n'-N(R(Rₑ) (A'2)

Formule dans laquelle :
▪ Y" représente le groupe -C(O)OH, -C(O)OZ", -CH₂-CH(OH)-SO₃H ou le groupe -CH₂-CH(OH)-SO₃-Z" ;
▪ R_{d} et Rₑ, indépendamment l'un de l'autre, représentent un radical alkyle ou hydroxyalkyle en C₁-C₄
▪ Z" représente un contre ion cationique issu d'un métal alcalin ou alcalinoterreux, tel que le sodium, un ion ammonium ou un ion issu d'une amine organique ;
▪ R_{a"} représente un groupe alkyle ou alcényle en C₁₀-C₃₀ d'un acide R_{a'}-C(O)OH de préférence présent dans l'huile de coprah ou dans l'huile de lin hydrolysée.
▪ n et n', indépendamment l'un de l'autre, désignent un nombre entier allant de 1 à 3.
▪ Parmi les composés de formule (A'2), on peut citer le composé classé dans le dictionnaire CTFA sous la dénomination sodium diethylaminopropyl cocoaspartamide et commercialisé par la société CHIMEX sous l'appellation CHIMEXANE HB

Parmi les agents tensioactifs amphotères ou zwittérioniques cités ci-dessus, on utilise de préférence les alkyl(C₈-C₂₀)bétaïnes telles que la cocobétaïne, les alkyl(C₈-C₂₀)amidoalkyl(C₃-C₈)bétaïnes telles que la cocamidopropylbétaïne, les composés de formule (B'2) tels que le sel de sodium du lauryl amino succinamate de diéthylaminopropyle (nom INCI) sodium diethylaminopropyl cocoaspartamide) et leurs mélanges.

De préférence, le ou les tensioactifs peu(ven)t être choisi(s) parmi les tensioactifs non ioniques, les tensioactifs amphotères ou zwiterrioniques et les tensioactifs cationiques, siliconés ou non.

### ETAPES b) ET c)

Le procédé de traitement cosmétique des fibres kératiniques selon l'invention, comporte notamment des étapes b) et c) consistant respectivement à appliquer une tension mécanique à ces fibres kératiniques et à les exposer sous tension mécanique à des micro-ondes à une pression allant de 50 000 à 250 000 Pa, mieux de 75 000 à 150 000 Pa, encore mieux à pression atmosphérique en présence d'au moins un solvant sous forme vapeur au contact desdites fibres kératiniques et sans qu'il y ait séchage complet des fibres kératiniques pendant la totalité de l'exposition aux micro-ondes.

En cas de séchage complet à l'étape c), la mise en forme n'est pas obtenue.

Par conséquent, notamment lorsque les étapes a), b) et c) sont effectuées dans cet ordre, une étape consistant à appliquer à nouveau une certaine quantité de la composition de l'étape a) ou une certaine quantité de solvant peut être effectuée juste avant l'étape c) (afin d'assurer le maintien de l'humidification des fibres kératiniques).

Il est entendu que lorsqu'une telle étape d'application d'une composition de l'étape a) est à nouveau effectuée avant l'étape c), ladite composition contient la même substance que celle qui avait été appliquée lors de l'étape a), c'est-à-dire :
- qu'elle contient au moins un tensioactif, ladite composition étant exempte d'hydroxydes alcalins ou alcalino-terreux à un pH supérieur à 12 ou d'agents réducteurs permettant la rupture des liaisons disulfures.

Lors de l'étape b) le rayonnement micro-onde peut déjà exister ou non, de même que le solvant sous forme vapeur. Autrement dit, l'étape b) est préalable ou simultanée à l'étape c).

Par « tension mécanique appliquée à ces cheveux », il faut comprendre une tension mécanique appliquée à une portion au moins de la longueur desdits cheveux.

Par « micro-ondes », il faut comprendre un rayonnement électromagnétique de fréquence comprise entre 500 MHz et 300 GHz.

La fréquence des micro-ondes utilisées lors de l'étape c) est préférentiellement comprise entre 500 MHz et 300 GHz, par exemple allant de 500 MHz à 10 GHz, en particulier de 915 MHz à 2,45 GHz.

La puissance des micro-ondes utilisées lors de l'étape c) peut aller de 100 à 2000 W, de préférence de 100 à 500 W, et plus particulièrement de 180 à 450W.

Les micro-ondes peuvent être générées par un générateur de micro-ondes, par exemple un générateur à état solide tel qu'un magnétron.

L'expression « sans qu'il y ait séchage complet des cheveux » signifie qu'après l'étape c) les cheveux présentent un toucher humide. Les cheveux peuvent ainsi conserver au moins 1 %, notamment au moins 2 %, voire 5 % du poids des composés liquides (solvants) présents, avant l'étape c), à leur contact, ces composés liquides s'ajoutant à l'humidité naturelle du cheveu avant traitement.

L'application de la tension mécanique peut être réalisée par un dispositif d'application d'une tension mécanique, ce dispositif pouvant être configuré pour induire sur les cheveux une flexion, une traction, une torsion et/ou une compression, par exemple. Le dispositif d'application d'une tension mécanique peut exercer des contraintes mécaniques simultanément sur une ou plusieurs mèches de cheveux.

Le dispositif de tension mécanique peut être par exemple un bigoudi.

Le solvant sous forme vapeur est entièrement généré par l'évaporation d'un composé présent, avant émission des micro-ondes, au contact des cheveux traités.

Les cheveux traités ne doivent jamais être totalement secs pendant toute la durée d'action des micro-ondes. Autrement dit, les cheveux doivent être toujours imprégnés par le solvant pendant ladite exposition.

Pour faciliter l'imprégnation, le solvant peut être pulvérisé au préalable.

L'étape c) du procédé selon l'invention peut avoir lieu au sein d'une enceinte ainsi éventuellement que l'étape b), en outre les étapes b) et c) peuvent être mises en oeuvre dans la même, unique enceinte. L'enceinte peut former un écran aux micro-ondes.

L'enceinte peut, durant le procédé selon l'invention, notamment durant l'étape c), contenir les cheveux à traiter et le dispositif d'application d'une tension mécanique.

Par « contenir les cheveux » il faut comprendre contenir les cheveux sur tout ou partie de leur longueur.

L'enceinte peut recouvrir les cheveux sur une longueur par exemple supérieure ou égale à 5 cm. Ainsi, une longueur d'au moins 5 cm de cheveux peut être traitée dans l'enceinte.

L'enceinte peut être immobile par rapport aux cheveux traités lors de l'émission des micro-ondes ou mobile par rapport aux cheveux, étant par exemple déplacée le long des cheveux à traiter.

Les micro-ondes peuvent être émises, le cas échéant, depuis une antenne.

Comme indiqué précédemment, l'enceinte peut être configurée pour ne pas libérer dans le milieu extérieur le solvant sous forme vapeur, ou n'en libérer qu'une faible quantité, grâce par exemple à un recyclage du solvant, le recyclage ayant par exemple lieu sous forme vapeur ou liquide, après condensation du solvant.

L'enceinte peut comporter un matériau configuré pour absorber le solvant sous forme vapeur. L'enceinte peut comporter une paroi froide sur laquelle le solvant se condense et/ou une bouche d'aspiration du solvant sous forme vapeur.

Ainsi, le procédé selon l'invention peut comporter, pendant et/ou après l'étape c), une étape de recollecte du solvant, par exemple sous forme vapeur et/ou liquide et/ou absorbée sur un matériau.

L'enceinte est avantageusement sensiblement étanche aux micro-ondes. Autrement dit, l'enceinte peut être configurée pour contenir les micro-ondes émises. L'étape c) peut donc avoir lieu au sein d'une enceinte étanche aux micro-ondes.

L'enceinte peut comporter au moins un joint d'un matériau électriquement conducteur, par exemple élastiquement déformable, permettant de faire écran aux micro-ondes utilisées lors de l'étape c) tout en laissant sortir les cheveux de l'enceinte si nécessaire. Le joint peut comporter par exemple une mousse chargée de particules électriquement conductrices, une brosse formée de poils électriquement conducteurs ou un peigne comportant des dents métalliques.

Lorsque l'enceinte est sous forme de casque, l'enceinte peut comporter un blindage électromagnétique au travers duquel les cheveux traités peuvent passer. Un tel blindage permet de traiter les cheveux de l'utilisateur tout en protégeant le crâne de ce dernier des micro-ondes émises.

Le blindage électromagnétique peut être formé par exemple par une grille ou un grillage métallique.

Le dispositif de traitement servant à mettre en oeuvre le procédé peut comporter un système d'avertissement sonore et/ou lumineux, par exemple pour avertir l'utilisateur d'une fuite de micro-ondes à l'extérieur de l'enceinte et/ou d'une température excessive à l'intérieur de l'enceinte. Le dispositif de traitement comporte avantageusement un système de sécurité empêchant l'émission des micro-ondes tant que l'enceinte n'est pas fermée et/ou en cas de fonctionnement anormal, par exemple de température excessive et/ou en l'absence de solvant.

Le dispositif de traitement peut être configuré pour contrôler la durée d'émission des micro-ondes, afin de ne pas atteindre une durée de traitement susceptible d'endommager les cheveux.

Le procédé selon l'invention peut comporter, avant l'étape c), une étape de détection de la fermeture de l'enceinte. Par exemple, un contacteur est actionné quand l'enceinte est fermée.

L'envoi des micro-ondes peut être conditionné à la détection de la fermeture de l'enceinte.

Le procédé selon l'invention peut, en outre, comporter une étape de détection de la mise en place des cheveux destinés à être traités, avant l'étape c). Cette étape de détection peut être réalisée par exemple par un capteur optique et/ou un palpeur mécanique.

Le procédé selon l'invention peut comporter, par exemple durant l'étape c), une étape de mesure de la température à laquelle sont soumis les cheveux traités. Cette étape de mesure de la température peut être réalisée par un thermomètre sans contact avec les cheveux.

L'enceinte, par exemple lorsqu'elle est définie par une pince, peut inclure tout ou partie du dispositif d'application de la tension mécanique.

Le dispositif d'application de la tension mécanique peut comporter un ou plusieurs bigoudi(s) ou autre dispositif d'enroulement, par exemple électriquement isolant(s) et compatible(s) avec une exposition aux micro-ondes, des mâchoires et/ou un ou plusieurs peignes.

Le dispositif de traitement peut être configuré pour permettre d'utiliser plusieurs dispositifs d'application de contrainte différents, servant par exemple à boucler les cheveux ou au contraire à les lisser. Les dispositifs peuvent être interchangeables par l'utilisateur.

Le dispositif de traitement peut être agencé pour reconnaître automatiquement le dispositif d'application de contrainte utilisé, le cas échéant, par exemple grâce à des contacts électriques ou à un ou plusieurs interrupteurs.

Le dispositif d'application de la tension mécanique peut être configuré de manière à placer les cheveux traités à plat durant l'exposition aux micro-ondes.

Quels que soient les exemples de réalisation considérés, les cheveux traités lors de l'étape b) peuvent être soumis à une ou à une pluralité de contraintes mécaniques. La/les contrainte(s) mécanique(s) peuvent être choisies parmi les contraintes de fléchissement, de redressement, de compression, de torsion et/ou de traction. Les contraintes appliquées peuvent viser à boucler les cheveux ou au contraire à les lisser. Les contraintes appliquées peuvent aussi viser à boucler les cheveux sur une portion de leur longueur et les lisser sur une autre portion de leur longueur.

Selon un mode de réalisation particulier de l'invention, l'étape b) est réalisée par application d'au moins une contrainte de torsion, de traction ou de compression sur les matières kératiniques.

Le dispositif de traitement peut comporter, au sein d'une même pièce à main, le générateur de micro-ondes ainsi que le dispositif d'application de la tension mécanique. Par « pièce à main » on désigne une pièce manipulée par l'utilisateur d'une main durant le traitement des cheveux.

Lorsque le dispositif de traitement comporte une pince, les micro-ondes peuvent être émises par seulement l'une des branches de la pince ou par l'ensemble des branches de la pince.

Comme moyens d'acheminement utilisables pour acheminer les micro-ondes depuis le générateur vers l'enceinte, on peut citer les guides d'onde, par exemple un câble coaxial flexible d'une longueur inférieure à 10 m, de préférence inférieure à 5 m, de diamètre inférieur à 5 cm, de préférence inférieur à 2 cm, et les ensembles comportant au moins une antenne émettrice de rayonnement électromagnétique et au moins une antenne réceptrice de rayonnement électromagnétique.

Le générateur de micro-ondes et/ou l'enceinte peu(ven)t être configuré(s) pour soumettre les cheveux traités durant l'étape c) à un rayonnement micro-onde variable dans sa distribution spatiale au sein de l'enceinte, par exemple tournant. Un rayonnement micro-onde tournant peut avantageusement permettre d'exposer de façon plus homogène les cheveux traités audit rayonnement et ainsi diminuer le risque d'une surexposition locale au rayonnement.

Le ou les solvants sont par exemple des liquides ayant un point d'ébullition inférieur à 200°C.

Ledit solvant peut par exemple être et est de préférence contenu dans la composition comprenant au moins un corps gras de l'étape a).

Ledit solvant peut comprendre, en particulier consister en un milieu liquide protique polaire ayant une constante diélectrique à 20°C qui est supérieure ou égale à 8, de préférence supérieure ou égale à 10 et en particulier supérieure ou égale à 15.

Selon un mode de réalisation préféré, les solvants utilisés comprennent de l'eau. Encore plus préférentiellement, le solvant utilisé est de l'eau.

Selon un autre mode de réalisation préféré, ce solvant est du propanol ou de l'isopropanol.

Selon encore un autre mode de réalisation préféré, les solvants sont constitués d'un mélange d'eau et de propanol ou d'isopropanol.

Dans un exemple de réalisation, le solvant sous forme vapeur peut être généré par chauffage direct du solvant à l'état liquide par les micro-ondes.

Le solvant sous forme vapeur peut avoir au voisinage et/ou au contact des cheveux, lors de l'étape c), une température comprise entre 80 et 200°C, de préférence entre 100 et 150°C, par exemple entre 120 et 150°C.

La pression à laquelle sont soumis les cheveux traités, lors de l'étape c), est proche de la pression atmosphérique, elle peut varier de 50 000 à 250 000 Pa, mieux de 75 000 à 150 000 Pa.

Les cheveux peuvent, pendant une partie ou la totalité de l'étape c), être présents dans un volume défini par au moins un mur d'un matériau, ledit matériau permettant aux micro-ondes de passer à travers le mur et limitant l'évaporation du composé présent, avant émission des micro-ondes, au contact des cheveux traités.

L'utilisation d'un tel matériau peut avantageusement limiter le séchage des cheveux pendant le traitement selon l'invention.

Ledit matériau peut comporter, en particulier consister en du cellophane et/ou avoir une faible porosité. Dans une variante, le matériau peut être poreux et en particulier être une maille.

Quels que soient les exemples de réalisation considérés, la durée de l'étape c) peut varier de 1 seconde à 60 minutes, de préférence de 1 seconde à 30 minutes, mieux de 30 secondes à 20 minutes, plus particulièrement de 3 à 15 minutes.

Quels que soient les exemples de réalisation considérés, l'étape c) peut être répétée par exemple entre 0 et 10 fois, préférentiellement entre 0 et 5 fois.

### DESCRIPTION DES FIGURES

L'invention pourra être mieux comprise à la lecture de la description détaillée qui va suivre, d'exemples non limitatifs de mise en oeuvre de celle-ci, et à l'examen du dessin annexé, sur lequel :
- les figures 1 à 5 représentent, de manière schématique et partielle, des exemples de réalisation de dispositifs de traitement selon l'invention, et
- les figures 6, 7 et 9 à 13 représentent des mèches de cheveux ayant subi divers traitements cosmétiques selon le procédé de l'invention ou selon un procédé hors invention.
- la figure 8 représente, de manière schématique et partielle, un exemple de réalisation selon l'invention.

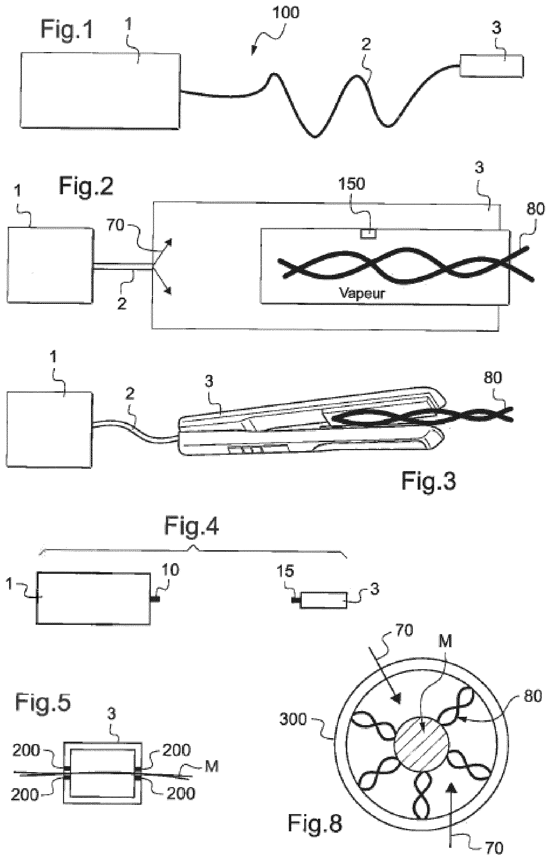

On a représenté à la figure 1 un dispositif de traitement 100 comportant une pièce à main 3 comportant une enceinte dans laquelle sont reçus les cheveux à traiter, reliée par un flexible 2 à un poste de base 1 comportant un générateur de micro-ondes.

Le flexible 2 peut comporter un guide d'ondes.

La figure 2 représente un détail de la figure 1.

Les micro-ondes 70 acheminées au sein de la pièce à main 3 par le guide d'onde 2 permettent de chauffer le solvant liquide présent sur les cheveux à traiter et de le faire passer à l'état de solvant sous forme vapeur. Dans cet exemple de réalisation, un dispositif d'application d'une tension mécanique ainsi qu'une mèche de cheveux (non représentés) sont présents au sein de la pièce à main 3 et les cheveux sont exposés à la fois aux micro-ondes et au solvant sous forme vapeur 80. Un détecteur de température 150 peut être présent afin de mesurer la température de la mèche de cheveux traitée et un système de contrôle, par exemple à micro-processeur, peut permettre d'interrompre ou moduler l'émission des micro-ondes en cas de température détectée supérieure à un seuil prédéfini.

A la figure 3 est représenté un exemple de réalisation où l'enceinte de traitement est formée par fermeture d'une pince constituant tout ou partie de la pièce à main 3. La pince permet, lorsqu'ouverte, d'introduire entre les branches une ou plusieurs mèche(s) de cheveux à traiter. Chaque branche définit par exemple la moitié de l'enceinte.

Les micro-ondes peuvent être émises par une seule ou par les deux branches de la pince.

Un capteur (non représenté) peut renseigner le dispositif de traitement sur le fait que la pince est fermée et l'envoi des micro-ondes peut être conditionné à la détection de cette fermeture.

La tension mécanique appliquée aux cheveux peut être une traction, afin de les lisser.

Dans tous les exemples qui précèdent, le moyen d'acheminement des micro-ondes peut comporter une antenne émettrice 10 et une antenne réceptrice 15, comme illustré à la figure 4.

On a, par ailleurs, représenté à la figure 5 une mèche de cheveux M présente dans une enceinte de traitement de la pièce à main 3. L'enceinte est étanche aux micro-ondes et comporte à cet effet, par exemple, des joints de mousse électriquement conductrice 200 réfléchissant le rayonnement micro-ondes là ou les cheveux quittent l'enceinte.

Dans une variante non illustrée, le générateur de micro-ondes 1 peut, par exemple, être présent au sein de l'enceinte et/ou du dispositif d'application d'une tension mécanique.

La figure 8 représente un exemple de réalisation dans lequel une mèche de cheveux M est présente dans un volume délimité par un mur 300 d'un matériau, ledit matériau permettant aux micro-ondes 70 de passer à travers le mur, et contenant la vapeur 80 générée.

Le contenant de la vapeur 80 permet avantageusement d'humidifier les cheveux M pendant le traitement.

Ledit matériau comprend, en particulier consiste en un film de cellophane. Dans une variante, le matériau peut être poreux et en particulier être une maille.

### ETAPES d) et e)

Le procédé selon l'invention peut, en outre, comporter au moins une étape supplémentaire de prétraitement d) et/ou une étape supplémentaire de post-traitement e), ces étapes consistant à effectuer sur les matières kératiniques au moins un traitement classique choisi parmi une coloration d'oxydation, une coloration directe, une décoloration, une déformation permanente à base d'un ou plusieurs réducteurs, par exemples thiolés ou à base d'un ou plusieurs hydroxydes alcalins ou alcalino-terreux, un soin, un masque et/ou un shampooing.

Avantageusement, la/les composition(s) appliquée(s) lors de(s) (l')étape(s) supplémentaire(s) d) est/sont exempte(s) de tensioactif.

Avantageusement, la/les composition(s) appliquée(s) lors de(s) (l')étape(s) supplémentaire(s) e) est/sont exempte(s) de tensioactif.

L'étape d) a lieu avant les étapes a), b) et c).

L'étape e) a lieu après les étapes a), b) et c).

Lorsque une étape supplémentaire de prétraitement d) et une étape supplémentaire de post-traitement e) sont mises en oeuvre, ces étapes peuvent être identiques ou différentes, de préférence, elles sont différentes.

Selon un mode de réalisation préféré de l'invention, l'étape supplémentaire est une étape de prétraitement d).

Lorsque l'étape supplémentaire est une étape comprenant une déformation permanente à base d'un ou plusieurs réducteurs, alors cette étape peut être suivie d'une étape comprenant l'application sur les cheveux d'au moins une composition de fixation comprenant un ou plusieurs agents oxydants.

Avantageusement, la/les composition(s) appliquée(s) lors de(s) (l')étape(s) supplémentaire(s) d) est/sont exempte(s) d'hydroxydes alcalins ou alcalino-terreux à un pH supérieur à 12 ou d'agents réducteurs permettant la rupture des liaisons disulfures.

Avantageusement, la/les composition(s) appliquée(s) lors de(s) (l')étape(s) supplémentaire(s) e) est/sont exempte(s) d'hydroxydes alcalins ou alcalino-terreux à un pH supérieur à 12 ou d' agents réducteurs permettant la rupture des liaisons disulfures.

La durée de l'étape d) peut varier selon les performances de mise en forme souhaitées et la nature des cheveux, par exemple.

La composition utilisée à l'étape a) ainsi que les éventuelles compositions utilisées aux étapes supplémentaires d) et e) peuvent être appliquées alors que les cheveux sont présents dans l'enceinte, par exemple grâce à un système d'application adapté. Le système d'application comporte par exemple un tampon, un peigne, un ou plusieurs orifices de distribution ou une buse de pulvérisation, disposé dans l'enceinte ou à l'extérieur de celle-ci, par exemple sur le trajet des cheveux sortant de ou entrant dans l'enceinte.

La composition utilisée à l'étape d) peut être soumise au rayonnement micro-onde.

Le dispositif de traitement peut comporter un capteur sensible à une caractéristique du cheveu, par exemple la couleur, la résistance mécanique, l'état de surface, l'humidité, et le dispositif de traitement peut contrôler au moins un paramètre du traitement en fonction de la caractéristique ainsi détectée, par exemple l'énergie des micro-ondes, la température du solvant, la durée du traitement et/ou la contrainte mécanique exercée.

Le présent brevet décrit un dispositif ne relevant pas de l'invention revendiquée, à savoir un dispositif de traitement des cheveux pour la mise en oeuvre du procédé tel que défini ci-dessus, comportant :
- un dispositif d'application d'une tension mécanique aux cheveux,
- un générateur de micro-ondes,
- au moins une composition comprenant au moins une substance choisie parmi les corps gras, les polymères siliconés et les tensioactifs.

Toutes les caractéristiques précisées à l'égard du procédé ci-dessus s'appliquent au dispositif de traitement.

Ainsi, le dispositif de traitement peut par exemple définir une enceinte de traitement formant écran aux micro-ondes.

Toutes les compositions utilisées dans le procédé selon l'invention peuvent se présenter indépendamment l'une de l'autre sous forme d'une lotion, épaissie ou non, d'une crème, d'un gel ou d'une mousse.

Les exemples suivants sont donnés à des fins d'illustration et sont non limitatifs de la présente invention.

### Exemples

### Exemple 1 : Procédé ne relevant pas de l'invention revendiquée pour boucler durablement les cheveux à l'aide d'une composition contenant au moins un corps gras

Une mèche de cheveux naturels raides de 1 g de 20 cm humidifiée a été traitée de la façon suivante.

La mèche est shampooinée et essorée puis une composition aqueuse comportant au moins un corps gras est appliquée avec un rapport de bain 2 pour 1 au minimum régulièrement le long de la mèche.

La mèche est ensuite enroulée et fixée autour d'un bigoudi.

Puis la mèche est placée dans un milieu confiné (type cellophane) ou non puis est traitée par l'émission de micro-ondes *via* un micro ondes ménager (SAMSUNG Combi CE 137nem ; 2,45GHz) pendant 15 minutes avec une puissance de 300W.

La fin du traitement est suivie d'un rinçage ou shampooing suivant le cas.

Le tableau qui suit renseigne sur la composition aqueuse appliquée.

| **Composants -** | **Quantité en pourcentage en poids (produit commercial en l'état)** |
|---|---|
| CETEARYL ALCOHOL (ECOROL 68/50 P de ECOGREEN OLEOCHEMICALS) | 5 |
| CETYL ESTERS (CRODAMOL MS-PA (MH) de CRODA) | 1 |
| BEHENTRIMONIUM CHLORIDE (VARISOFT BT 85 (FLAKED)de EVONIK GOLDSCHMIDT) | 1 |
| AMODIMETHICONE (and) TRIDECETH-6 (and) CETRIMONIUM CHLORIDE (XIAMETER MEM-8299 EMULSION de DOW CORNING) | 1,5 |
| Eau | QSP 100 |

Ce procédé permet une mise en forme de la mèche (initialement raide), la mèche obtenue est durablement bouclée, on observe également un gain du volume de la mèche.

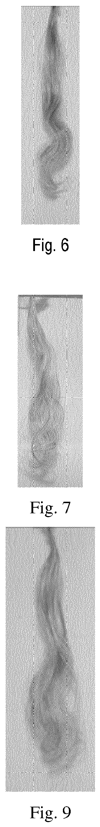

Le résultat est reporté en figure 6.

### Exemple 2 : Procédé ne relevant pas de l'invention revendiquée pour boucler durablement les cheveux à l'aide d'une composition contenant au moins un corps gras

Une mèche de cheveux naturels raides de 1 g de 20 cm humidifiée a été traitée de la façon similaire à l'exemple 1, à la différence près que la composition aqueuse appliquée ici est celle détaillée dans le tableau suivant.

| **Composants-Nom INCI** | **Quantité en pourcentage en poids (produit commercial en l'état)** |
|---|---|
| BEESWAX (cire d'abeille) (CIRE D'ABEILLE BLANCHE GR B 889 de KOSTER KEUNEN) | 1,5 |
| MINERAL OIL (and) MICROCRYSTALLINE WAX (and) PARAFFIN (VASELINE BLANCHE CODEX 236 de AIGLON) | 6,5 |
| MINERAL OIL (BLANDOL de SONNEBORN) | 16 |
| CETEARYL ALCOHOL (ECOROL 68/50 P de ECOGREEN OLEOCHEMICALS) | 2 |
| PROPYLENE GLYCOLUSP/EP de DOW CHEMICAL) | 5 |
| Eau | QSP 100 |

Ce procédé permet une mise en forme de la mèche, la mèche obtenue est durablement bouclée, on observe également un gain du volume de la mèche.

Le résultat est reporté en figure 7.

### Exemple 3 : Procédé ne relevant pas de l'invention revendiquée pour boucler durablement les cheveux à l'aide d'une composition contenant au moins un polymère non siliconé

Une mèche de cheveux naturels raides de 1g de 20 cm humidifiée a été traitée de la façon suivante.

La mèche est shampooinée et essorée puis une composition aqueuse comportant au moins un polymère non siliconé est appliquée avec un rapport de bain 2 pour 1 au minimum régulièrement le long de la mèche.

La mèche est ensuite enroulée et fixée autour d'un bigoudi.

Puis la mèche est placée dans un milieu confiné (type cellophane) puis est traitée par l'émission de micro-ondes *via* un micro ondes ménager (SAMSUNG Combi CE 137nem ; 2,45GHz) pendant 5 minutes avec une puissance de 450 W.

La fin du traitement est suivie d'un rinçage ou shampooing suivant le cas.

Le tableau qui suit renseigne sur la composition aqueuse appliquée.

| **Composants** | **Quantité en pourcentage en poids (produit commercial en l'état)** |
|---|---|
| POTATO STARCH MODIFIED (STRUCTURE SOLANACE de AKZO NOBEL) | 0,3 |
| CARBOMER (CARBOPOL ULTREZ 10 POLYMER de LUBRIZOL) | 0,45 |
| HYDROXYPROPYL GUAR (JAGUAR HP 105 de RHODIA) | 0,25 |
| POLYQUATERNIUM-4 (CELQUAT LOR de AKZO NOBEL) | 0,3 |
| BEHENTRIMONIUM CHLORIDE (VARISOFT BT 85 (FLAKED) de EVONIK GOLDSCHMIDT) | 0,5 |
| CETYL ALCOHOL (and) BEHENTRIMONIUM METHOSULFATE (and) QUATERNIUM-33 (CUTISSENTIAL BEHENYL 18MEA-PA-(MH) de CRODA) | 0,1 |
| PROPYLENE GLYCOL (PROPYLENE GLYCOL USP/EP de DOW CHEMICAL) | 2,5 |
| 2-OLEAMIDO-1,3-OCTADECANEDIOL (MEXANYL GZ de CHIMEX) | 0,01 |
| QUATERNIUM-87 (VARISOFT W 575 PG N de EVONIK GOLDSCHMIDT) | 0,05 |
| CYCLOPENTASILOXANE (and) DIMETHICONOL (XIAMETER PMX-1501 FLUID de DOW CORNING) | 6 |
| PEG/PPG-17/18 DIMETHICONE | 0,5 |

| **Composants** | **Quantité en pourcentage en poids (produit commercial en l'état)** |
|---|---|
| AMODIMETHICONE (and) TRIDECETH-6 (and) CETRIMONIUM CHLORIDE | 0,25 |
| TRIETHANOLAMINE | 0,6 |
| Eau | QSP 100 |

Ce procédé permet une mise en forme de la mèche (initialement raide), la mèche obtenue est durablement bouclée, on observe également un gain du volume de la mèche.

Le résultat est reporté en figure 9.

### Exemple 4 : Procédé ne relevant pas de l'invention revendiquée pour boucler durablement les cheveux à l'aide d'une composition contenant au moins un polymère non siliconé

Une mèche de cheveux naturels raides de 1g de 20 cm humidifiée a été traitée de la façon similaire à l'exemple 1, au moyen de la composition aqueuse détaillée dans le tableau suivant.

| **Composants** | **Quantité en pourcentage en poids (produit commercial en l'état)** |
|---|---|
| METHYLISOTHIAZOLINONE (NEOLONE 950 PRESERVATIVE de ROHM AND HAAS (DOW CHEMICAL)) | 0,1 |
| CAPRYLYL GLYCOL (DERMOSOFT OCTIOL de DR STRAETMANS) | 0,4 |
| ACRYLATES/C10-30 ALKYL ACRYLATE CROSSPOLYMER (PEMULEN TR-1 POLYMER de LUBRIZOL) | 1,4 |
| VP/VA COPOLYMER ⁽LUVISKOL VA 64 W de BASF) | 10 |
| VP/DIMETHYLAMINOETHYLMETHACRYLATE | 12 |

| **Composants** | **Quantité en pourcentage en poids (produit commercial en l'état)** |
|---|---|
| COPOLYMER ⁽COPOLYMER 845-O de ISP (ASHLAND)) | |
| PEG-40 HYDROGENATED CASTOR OIL (EUMULGIN HRE 40 de COGNIS (BASF)) | 1 |
| NIACINAMIDE (NIACINAMIDE USP de LONZA) | 0,1 |
| PANTHENOL (DEXPANTHENOL de DAIICHI FINE CHEMICAL) | 0,1 |
| WATER | QSP 100 |

Ce procédé permet une mise en forme de la mèche, la mèche obtenue est durablement bouclée, on observe également un gain du volume de la mèche.

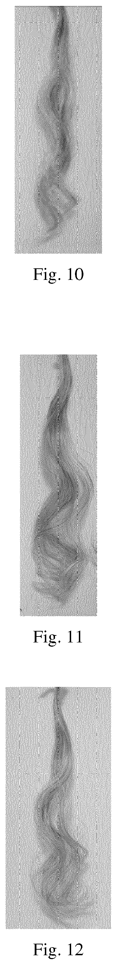

Le résultat est reporté en figure 10.

### Exemple 5 : Procédé ne relevant pas de l'invention revendiquée pour boucler durablement les cheveux à l'aide d'une composition contenant au moins un polymère non siliconé

Une mèche de cheveux naturels raides de 1g de 20 cm humidifiée a été traitée de la façon similaire à l'exemple 1, au moyen de la composition aqueuse détaillée dans le tableau suivant.

| **Composants** | **Quantité en pourcentage en poids (produit commercial en l'état)** |
|---|---|
| ETHANOLAMINE (MONOETHANOLAMINE CARE de BASF) | 0,47 |
| OLETH-30 (EUMULGIN O 30 de COGNIS (BASF)) | 4,5 |
| LAURETH-12 (REWOPAL 12 de EVONIK GOLDSCHMIDT) | 6,3 |
| DECETH-5 (EUMULGIN BL 589 de COGNIS (BASF)) | 4,5 |
| DECETH-3 (EUMULGIN BL 309 de COGNIS (BASF)) | 17,2 |
| OLEYL ALCOHOL (HD OCENOL 80/85V de COGNIS (BASF)) | 1,8 |
| TRIDECETH-2 CARBOXAMIDE MEA (AMIDET A15/LAO 55 de KAO) | 4 |
| HEXADIMETHRINE CHLORIDE (MEXOMERE PO de CHIMEX) | 3 |
| POLYQUATERNIUM-22 (MERQUAT 280 POLYMER de NALCO (LUBRIZOL)) | 3 |
| GLYCERIN (GLYCERIN 99,8% PF de EMERY OLEOCHEMICALS) | 3 |
| AMMONIUM THIOLACTATE (THIOLACTATE D'AMMONIUM 58% ( 50% TLA ) de BRUNO BOCK) | 0,8 |
| WATER | QSP 100 |

Ce procédé permet une mise en forme de la mèche, la mèche obtenue est durablement bouclée, on observe également un gain du volume de la mèche.

Le résultat est reporté en figure 11.

### Exemple 6 : Procédé selon l'invention pour boucler durablement les cheveux à l'aide d'une composition contenant au moins un tensioactif

Une mèche de cheveux naturels raides de 1g de 20 cm humidifiée a été traitée de la façon suivante.

La mèche est shampooinée et essorée puis une composition aqueuse conforme à l'invention comportant au moins un tensioactif est appliquée avec un rapport de bain 2 pour 1 au minimum régulièrement le long de la mèche.

La mèche est ensuite enroulée et fixée autour d'un bigoudi.

Puis la mèche est placée dans un milieu confiné (type film de cellophane), puis est traitée par l'émission de micro-ondes *via* un micro ondes ménager (SAMSUNG Combi CE 137nem, 2,5GHz) pendant 5 minutes avec une puissance de 450 W.

La fin du traitement est suivie d'un rinçage ou shampooing suivant le cas.

Le tableau qui suit renseigne sur la composition aqueuse appliquée.

| **Composants** | **Quantité en pourcentage en poids (produit commercial en l'état)** |
|---|---|
| DECETH-3 (EUMULGIN BL 309 de COGNIS (BASF)) | 22 |
| DECETH-5 (EUMULGIN BL 589 de COGNIS (BASF)) | 19,5 |
| COCAMIDE MIPA (and) ISOPROPANOLAMINE (REWOMID V 3203 de EVONIK GOLDSCHMIDT) | 7,2 |
| PROPYLENE GLYCOL (USP/EP de DOW CHEMICAL) | 15 |
| HEXYLENE GLYCOL de RHODIA | 1 |
| HYDROXYETHYL OLEYL DIMONIUM CHLORIDE en solution aqueuse à 30% | 3,33 |
| SODIUM METABISULFITE de BASF | 0,455 |
| ERYTHORBIC ACID de ZHENGZOU TUOYANG BIO ENGINEERING | 0,31 |
| Eau | QSP 100 |

Ce procédé permet une mise en forme de la mèche (initialement raide), la mèche obtenue est durablement bouclée, on observe également un gain du volume de la mèche.

Le résultat est reporté en figure 12.

### Exemple 7 : Procédé selon l'invention pour boucler durablement les cheveux à l'aide d'une composition contenant au moins un tensioactif

Une mèche de cheveux naturels raides de 1g de 20 cm humidifiée a été traitée de la façon similaire à l'exemple 1, à la différence près que la composition aqueuse appliquée ici est celle détaillée dans le tableau suivant.

| **Composants** | **Quantité en pourcentage en poids (produit commercial en l'état)** |
|---|---|
| POTATO STARCH MODIFIED (STRUCTURE SOLANACE de AKZO NOBEL) | 0,3 |
| CARBOMER (CARBOPOL ULTREZ 10 POLYMER de LUBRIZOL) | 0,38 |
| HYDROXYPROPYL GUAR (JAGUAR HP 105 de RHODIA) | 0,25 |
| POLYQUATERNIUM-4 (CERESIN WAX SP 254P de STRAHL & PITSCH) | 0,3 |
| BEHENTRIMONIUM CHLORIDE (VARISOFT BT 85 (FLAKED) de EVONIK GOLDSCHMIDT) | 0,5 |
| PROPYLENE GLYCOL(USP/EP de DOW CHEMICAL) | 2,5 |
| CYCLOPENTASILOXANE (and) DIMETHICONOL (XIAMETER PMX-1501 FLUID de DOW CORNING) | 10,6 |
| PEG/PPG-17/18 DIMETHICONE (XIAMETER OFX-5220 FLUID de DOW CORNING | 0,5 |
| Eau | QSP 100 |

Ce procédé permet une mise en forme de la mèche, la mèche obtenue est durablement bouclée, on observe également un gain du volume de la mèche.

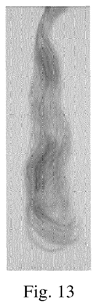

Le résultat est reporté en figure 13.

## Revendications

1. Procédé de traitement cosmétique des fibres kératiniques, de préférence les cheveux, comprenant au moins les étapes consistant à :
a) appliquer, sur lesdites fibres kératiniques, une composition contenant au moins une substance choisie parmi les tensioactifs,
b) appliquer une tension mécanique auxdites fibres kératiniques, et
c) exposer lesdites fibres kératiniques sous tension mécanique à des micro-ondes, à une pression allant de 50 000 à 250 000 Pa, en présence d'au moins un solvant sous forme vapeur au contact des fibres kératiniques et sans qu'il y ait séchage complet des fibres kératiniques pendant la totalité de l'exposition aux micro-ondes,
le ou les solvants sous forme vapeur étant entièrement générés par l'évaporation d'au moins un composé présent, avant émission des micro-ondes, au contact des fibres kératiniques,
l'étape a) ayant lieu préalablement à l'étape c),
**caractérisé en ce que** ladite composition est exempte d'hydroxydes alcalins ou alcalino-terreux à un pH supérieur à 12 ou d'agents réducteurs permettant la rupture des liaisons disulfures.

2. Procédé selon la revendication 1, dans lequel la pression va de 75 000 à 150 000 Pa, de préférence, la pression est la pression atmosphérique.

3. Procédé selon l'une quelconque des revendications 1 ou 2, dans lequel le ou lesdits tensioactif(s) sont choisi(s) parmi les tensioactifs non ioniques, les tensioactifs anioniques, les tensioactifs cationiques et les tensioactifs amphotères ou zwiterrioniques.

4. Procédé selon l'une quelconque des revendications précédentes, dans lequel la composition de l'étape a) est une composition aqueuse, comprenant de préférence de 20 à 99,9 % en poids d'eau par rapport au poids total de ladite composition.

5. Procédé selon l'une quelconque des revendications précédentes, dans lequel l'étape a) est préalable à l'étape b).

6. Procédé selon l'une quelconque des revendications précédentes dans lequel l'étape b) est réalisée par application d'au moins une contrainte de torsion, de traction ou de compression sur les matières kératiniques.

7. Procédé selon l'une quelconque des revendications précédentes dans lequel la durée de l'étape c) varie de 1 seconde à 60 minutes, de préférence de 1 seconde à 30 minutes, mieux de 30 secondes à 20 minutes, plus particulièrement de 3 à 15 minutes.

8. Procédé selon l'une quelconque des revendications précédentes, dans lequel les solvants utilisés comprennent de l'eau, plus préférentiellement, le solvant utilisé est de l'eau.

9. Procédé selon l'une quelconque des revendications précédentes comportant au moins une étape supplémentaire de prétraitement d) et/ou une étape supplémentaire de post-traitement e), ces étapes consistant à effectuer sur les matières kératiniques au moins un traitement classique choisi parmi une coloration d'oxydation, une coloration directe, une décoloration, une déformation permanente à base d'un ou plusieurs réducteurs, par exemples thiolés et/ou à base d'un ou plusieurs hydroxydes alcalins ou alcalino-terreux, un soin, un masque et/ou un shampooing.

## Patentansprüche

1. Verfahren zur kosmetischen Behandlung von Keratinfasern, vorzugsweise Haaren, das zumindest die aus Folgendem bestehenden Schritte umfasst:
a) ein Aufbringen einer Zusammensetzung, die mindestens eine aus Tensiden ausgewählte Verbindung enthält, auf die Keratinfasern,
b) ein Einwirkenlassen einer mechanischen Spannung auf die Keratinfasern und
c) ein Exponieren der Keratinfasern unter einer mechanischen Spannung gegenüber Mikrowellen bei einem Druck von 50 000 bis 250 000 Pa in Gegenwart mindestens eines dampfförmigen Lösungsmittels in Kontakt mit den Keratinfasern und ohne dass während der gesamten Exposition gegenüber den Mikrowellen ein vollständiges Trocknen der Keratinfasern erfolgt,
wobei das oder die dampfförmige(n) Lösungsmittel vollständig durch ein Verdampfen mindestens einer Verbindung gebildet wird bzw. werden, die vor dem Ausstrahlen von Mikrowellen in Kontakt mit den Keratinfasern vorhanden ist,
wobei Schritt a) vor Schritt c) erfolgt,
**dadurch gekennzeichnet, dass** die Zusammensetzung frei von Alkali- oder Erdalkalihydroxiden mit einem pH-Wert von mehr als 12 oder Reduktionsmitteln ist, die ein Brechen von Disulfidbindungen ermöglichen.

2. Verfahren nach Anspruch 1, wobei der Druck von 75 000 bis 150 000 Pa beträgt, der Druck vorzugsweise Atmosphärendruck ist.

3. Verfahren nach einem der Ansprüche 1 oder 2, wobei das Tensid oder die Tenside aus nichtionischen Tensiden, anionischen Tensiden, kationischen Tensiden und amphoteren oder zwitterionischen Tensiden ausgewählt ist bzw. sind.

4. Verfahren nach einem der vorhergehenden Ansprüche, wobei die Zusammensetzung von Schritt a) eine wässrige Zusammensetzung ist, die vorzugsweise von 20 bis 99,9 Gew.-% Wasser, bezogen auf das Gesamtgewicht der Zusammensetzung, umfasst.

5. Verfahren nach einem der vorhergehenden Ansprüche, wobei Schritt a) vor Schritt b) erfolgt.

6. Verfahren nach einem der vorhergehenden Ansprüche, wobei Schritt b) durch das Einwirkenlassen mindestens einer Torsions-, Zug- oder Kompressionsbeanspruchung auf die Keratinsubstanzen erfolgt.

7. Verfahren nach einem der vorhergehenden Ansprüche, wobei die Dauer von Schritt c) von 1 Sekunde bis 60 Minuten, vorzugsweise von 1 Sekunde bis 60 Minuten, noch mehr bevorzugt von 30 Sekunden bis 20 Minuten, insbesondere von 3 bis 15 Minuten, variiert.

8. Verfahren nach einem der vorhergehenden Ansprüche, wobei die verwendeten Lösungsmittel Wasser umfassen, wobei es sich bei dem verwendeten Lösungsmittel stärker bevorzugt um Wasser handelt.

9. Verfahren nach einem der vorhergehenden Ansprüche, das mindestens einen zusätzlichen Vorbehandlungsschritt d) und/oder einen zusätzlichen Nachbehandlungsschritt e) umfasst, wobei diese Schritte darin bestehen, an den Keratinsubstanzen mindestens eine klassische Behandlung durchzuführen, die aus einer Oxidationsfärbung, Direktfärbung, einem Bleichen, einer bleibenden Verformung auf der Grundlage eines oder mehrerer Reduktionsmittel, beispielsweise Thiolen und/oder auf der Grundlage eines oder mehrerer Alkali- oder Erdalkalihydroxide, einer Pflege, einer Maske und/oder einem Shampoo ausgewählt ist.

## Claims

1. Cosmetic process for treating keratin fibres, preferably the hair, comprising at least the steps consisting in:
a) applying to said keratin fibres a composition containing at least one substance chosen from surfactants,
b) applying a mechanical tension to said keratin fibres, and
c) exposing said keratin fibres under mechanical tension to microwaves, at a pressure ranging from 50 000 to 250 000 Pa, in the presence of at least one solvent in vapour form on contact with the keratin fibres and without there being complete drying of the keratin fibres throughout the entire exposure to the microwaves,
the solvent(s) in vapour form being entirely generated by evaporating at least one compound present, before emission of the microwaves, on contact with the keratin fibres,
step a) taking place prior to step c),
**characterized in that** said composition is free of alkali metal or alkaline-earth metal hydroxides having a pH above 12 or of reducing agents for cleaving the disulfide bonds.

2. Process according to Claim 1, in which the pressure ranges from 75 000 to 150 000 Pa, and, preferably, the pressure is atmospheric pressure.

3. Process according to either one of Claims 1 and 2, in which said surfactant(s) is (are) chosen from nonionic surfactants, anionic surfactants, cationic surfactants and amphoteric or zwitterionic surfactants.

4. Process according to any one of the preceding claims, in which the composition from step a) is an aqueous composition, preferably comprising from 20% to 99.9% by weight of water relative to the total weight of said composition.

5. Process according to any one of the preceding claims, in which step a) is prior to step b).

6. Process according to any one of the preceding claims, in which step b) is performed by applying at least one torsional, tensile or compressive stress on the keratin materials.

7. Process according to any one of the preceding claims, in which the duration of step c) ranges from 1 second to 60 minutes, preferably from 1 second to 30 minutes, better still from 30 seconds to 20 minutes and more particularly from 3 to 15 minutes.

8. Process according to any one of the preceding claims, in which the solvents used comprise water, and, more preferentially, the solvent used is water.

9. Process according to any one of the preceding claims, comprising at least one additional pretreatment step d) and/or one additional post-treatment step e), these steps consisting in performing on the keratin materials at least one standard treatment chosen from oxidation dyeing, direct dyeing, bleaching, permanent reshaping based on one or more reducing agents, for example thiol reducing agents, and/or based on one or more alkali metal or alkaline-earth metal hydroxides, a care treatment, a mask and/or a shampoo.
